(19) **Europäisches Patentamt** / **European Patent Office** / **Office européen des brevets**

(11) **EP 3 310 930 B1**

(12) **EUROPEAN PATENT SPECIFICATION**

(45) Date of publication and mention
of the grant of the patent:
**15.05.2019 Bulletin 2019/20**

(21) Application number: **16794331.5**

(22) Date of filing: **10.11.2016**

(51) Int Cl.:
*C12Q 1/6823* (2018.01)    *C12Q 1/6827* (2018.01)

(86) International application number:
**PCT/EP2016/077267**

(87) International publication number:
**WO 2017/081152 (18.05.2017 Gazette 2017/20)**

(54) **DIAGNOSTIC METHODS AND DEVICES**

DIAGNOSEVERFAHREN UND VORRICHTUNGEN

PROCÉDÉS ET DISPOSITIFS DE DIAGNOSTIC

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.11.2015 EP 15193960**

(43) Date of publication of application:
**25.04.2018 Bulletin 2018/17**

(73) Proprietors:
• **Administración General De La Communidad
Autónoma
De Euskadi
01010 Vitoria-Gasteiz (Araba/Álava) (ES)**
• **Universidad Del Pais Vasco
Euskal Herriko Unibertsitatea
48940 Leioa (Vizcaya) (ES)**
• **Basque Center for Macromolecular Design and
Engineering, POLYMAT Fundazioa
20018 Gipuzkoa (ES)**

(72) Inventors:
• **LAWRIE, Charles, Henderson
20014 San Sebastián (ES)**
• **BASABE-DESMONTS, Lourdes
48940 Leioa-Vizcaya (ES)**
• **SCHÄFER, Thomas
20018 Gipuzkoa (ES)**
• **LIZ MARZÁN, Luis, Manuel
2009 Gipuzkoa (ES)**
• **PARAK, Wolfgang, Johann
2009 Gipuzkoa (ES)**

(74) Representative: **Mewburn Ellis LLP
City Tower
40 Basinghall Street
London EC2V 5DE (GB)**

(56) References cited:
EP-A1- 1 867 990         WO-A1-82/02772
WO-A1-90/02205           WO-A1-99/23258
WO-A2-2004/053105        US-A1- 2004 180 369

• MINH-PHUONG NGOC BUI ET AL: "Gold
nanoparticle aggregation-based highly sensitive
DNA detection using atomic force microscopy",
ANALYTICAL AND BIOANALYTICAL
CHEMISTRY, SPRINGER, BERLIN, DE, vol. 388,
no. 5-6, 30 May 2007 (2007-05-30), pages
1185-1190, XP019537534, ISSN: 1618-2650, DOI:
10.1007/S00216-007-1354-4
• MARIA SANROMÁN-IGLESIAS ET AL:
"Conjugated Polymers As Molecular Gates for
Light-Controlled Release of Gold Nanoparticles",
ACS APPLIED MATERIALS AND INTERFACES,
vol. 7, no. 29, 29 July 2015 (2015-07-29), pages
15692-15695, XP055330123, US ISSN: 1944-8244,
DOI: 10.1021/acsami.5b05087 cited in the
application
• GOVOROV ET AL: "Generating heat with metal
nanoparticles", NANO TODAY, ELSEVIER,
AMSTERDAM, NL, vol. 2, no. 1, 1 February 2007
(2007-02-01), pages 30-38, XP005929428, ISSN:
1748-0132, DOI: 10.1016/S1748-0132(07)70017-8

## Description

### Field of the invention

The present invention relates to methods and devices employing a signal amplification step based on nanoparticle technology for the detection of bioanalytes, e.g. disease biomarkers.

### Background to the invention

The present invention is directed at methods and related products and kits for the detection of bioanalytes such as disease biomarkers e.g. genetic mutations or non-host genetic material.

Cancer is the second most common cause of death in the EU, responsible for around 1.75 million deaths in 2012 alone, with an estimated 3.45 million EU citizens diagnosed as having the condition in the same year. The gold standard of cancer diagnostics is the histological examination of tissue; however, such procedures are expensive, not without risk to the patient, and require consistent evaluation by expert pathologists. As a result, there has been a great deal of interest in the development of technologies to detect non-invasive biomarkers for the diagnosis and management of cancer. Of particular interest in this respect are technologies to detect mutated DNA sequences, as these have both diagnostic utility and can predict prognosis and responsiveness to targeted therapies. Biomarkers of this type are of significant scientific and clinical value in the practise of medicine generally, being useful in diagnosis, prognosis, screening, and risk assessment of a whole spectrum of diseases and disorders. Application of biomarkers in these ways is already being integrated into many healthcare systems, to achieve personalisation of treatment and disease prevention in the increasingly important move towards "personalised medicine".

Despite this, currently available technologies allowing for the detection of such biomarkers are limited, in that they require expensive equipment, are labour-intensive, and have long turn-around times. Moreover, the majority of commercially available technologies depend upon prior manipulation and amplification of the target DNA, as they are insufficiently sensitive for detection in a raw sample which may be of limited volume. As a result, their use is generally restricted to specialised laboratories.

Noble metal nanoparticles have a number of unique physiochemical properties, which has prompted investigation of their use in the development of new biosensing platforms. While there are many prior-described examples of methods and devices which exploit the unique properties of noble metal nanoparticles in the pursuit of more specific and sensitive biomolecular diagnostic tools (see Doria et al., Sensors, 2012, Vol. 12, pp 1657-1687 for a comprehensive review), the majority of these still require sample manipulation and amplification of the target DNA prior to detection.

WO 2005/118870 describes a method for detecting a target DNA sequence of interest using a micro-electrode array of immobilised probe oligonucleotides and a plurality of nanoparticles functionalised with probes for a second region of the target DNA. Target DNA in a test sample will bind to the electrode by the immobilised probes, which will in turn result in the aggregation of nanoparticles via the captured DNA. This aggregation can be detected as changes in the electrochemical impedance at the electrode site.

WO 2007/044025 describes a method for detecting a target DNA sequence of interest using nanoparticles functionalised with probes specific for two different regions of the target DNA. Target DNA in a test sample will result in the formation of complexes of gold nanoparticles which can be detected by measuring changes in the light scattering properties of the sample.

Sanromán-Iglesias et al., ACS Appl. Mater. Interfaces, 2015, Vol. 7, pp. 15692-15695, describes conjugated polymers as molecular gates for light-controlled release of gold nanoparticles. The remote release of nanoparticles into aqueous solution by photodegradation of a thin polymer allowed particle release to be detected by the naked eye.

Sanromán-Iglesias et al., ACS Sens., 2016, Vol. 1, pp. 1110-1116, describes detection of a single nucleotide polymorphism (SNP) by selective aggregation of nanoparticles. The effect of nanoparticle size on the sensitivity limit of nanoparticle biosensors in the discrimination of SNP was investigated. A 5-fold increase in particle size, at constant gold concentration, lead to an improvement in the limit of detection by 3 orders of magnitude.

Zhou et al., J Am Chem Soc, 2010, Vol. 26, pp 6932-6934 describe a method for the detection of a target DNA sequence of interest which incorporates a biomineralisation amplification process. In this method, an array of immobilised probe oligonucleotides are provided alongside a plurality of nanoparticles functionalised with both probes for a second region of the target DNA and a biomineralisation capable silicatein. Target DNA in a test sample will bind to the immobilised probes, which will in turn result in the aggregation of nanoparticles via the captured DNA. The nanoparticle bound silicatein then catalyses the synthesis of silica, which can entrap a second gold nanoparticle species to produce detectable changes in the light scattering properties of the sample. A further silver staining step can also be employed to allow detection of the sequence of interest at concentrations as low as 50 aM.

Bui et al. (Anal Bioanal Chem (2007) 388:1185-1190) describe detection of DNA functionalised on gold nanoparticles, using atomic force microscopy.

EP 1867990 relates to a device and a method for amplifying a signal generated from primary nanoparticle labels in an assay.

WO 82/02772 describes a method, composition, kit and system for carrying out an immunoassay for a particular biological analyte. Govorov et al. (Nanotoday (2007) Vol 2 Number 1:30-38) describe the photothermal effects of metal colloidal nanoparticles in the presence of electromagnetic radiation.

US 2004/0180369 discloses a nucleic acid hybridization detection assay, using capture proves comprising single-stranded oligonucleotides immobilized to a solid substrate surface.

WO 90/02205 relates to a method of detecting, identifying and/or quantitating nucleic acid in a sample through determination of agglutination of suspendable particles having a member of a specific binding pair bound thereto.

WO 99/23258 discloses methods and compositions for detecting a specified nucleic acid sequence in a sample by optically detecting formation, or inhibition of formation, of a hybridization complex. There remains a need for methods and kits for the detection of disease biomarkers (such as genetic mutations) in a low cost, rapid, and highly sensitive manner. Portability and ease-of-use are also desired. In particular, a method which avoids the need for sample manipulation and amplification of the target DNA prior to detection is desired. Moreover, there is a need for methods and kits allowing for the detection of disease biomarkers while providing convenient, on-device readout. The present invention addresses these and other needs, in a system that can be quickly and easily adapted to the detection of various target bioanalytes, and in a variety of biological or environmental test samples.

## Brief Description of the Invention

The present invention provides methods and devices for the detection of target analytes in a test sample. Broadly, the invention makes use of a signal amplification method in which the release of a readily detectable signal is conditional upon target analyte-induced agglomeration of nanoparticles.

Accordingly, in a first aspect the present invention provides a method for detecting the presence or absence of a target analyte in a test sample, comprising the steps of:

providing a plurality of detector nanoparticles comprising a first detector nanoparticle functionalised with a first probe specific for a first region of a target analyte and a second detector nanoparticle functionalised with a second probe specific for a second region of said target analyte; and

contacting said detector nanoparticles with a test sample under conditions suitable for the binding of the specific probes to the target analyte, wherein target analyte-induced agglomeration of the detector nanoparticles permits the release of a detectable signal means from a signal reservoir by breakdown of a partition closing the signal reservoir, and wherein the partition is a thermoresponsive polymer layer and the agglomerated detector nanoparticles direct a heat transfer sufficient to cause at least partial thermal breakdown of said thermoresponsive polymer layer, thereby releasing the detectable signal means, and wherein the heat transfer is triggered by irradiation of the agglomerated detector nanoparticles with a source of electromagnetic radiation.

In this way, a sensitive and adaptable detection event is conditionally linked to a signal event; this signal event may be more readily discernible than the detection event, thus allowing for the detection of analytes of interest without the need for prior sample manipulation or amplification. Linking a detection event to a signal event in this way also allows for the method to be readily adaptable to the detection of a variety of analytes, simply by changing the detector nanoparticle probes.

In some cases in accordance with this and other aspects of the invention, the target analyte may be a nucleic acid, a protein or other biological molecule. The first and second probes a chosen so as to be specific for (i.e. exhibit selective binding to) the target analyte. In cases where the target analyte comprises a nucleic acid, the first and/or second probes may be oligonucleotides having complementary sequence to (respective portions of) the target analyte sequence. In cases where the target analyte comprises a protein (glycosylated or otherwise), the first and second probes may for example each take the form of antibody molecules (e.g. monoclonal antibody, FAb, scFV) that selectively binds to a respective epitope present on the protein. In a similar fashion the first and second probes may take the form of aptamers that selectively bind to the target analyte. By virtue of the first and second probes binding to different regions (e.g. different epitopes) on the target analyte, a sandwich or bridging binding arrangement is brought about, leading to analyte-mediated agglomeration of the detector nanoparticles.

According to the present invention, release of the detectable signal means is via breakdown of a partition closing the signal reservoir. This partition is a thermoresponsive polymer layer which may be broken down by a heat transfer directed by agglomerates of the detector nanoparticles. For example, the signal reservoir may comprise one or more microspheres comprising a shell, e.g. a shell formed of a thermoresponsive polymer, enclosing a core that comprises the detectable signal means. In this way the shell of the microsphere provides the partition. According to the present invention, this heat transfer is triggered by irradiation of the agglomerated detector nanoparticles with a source of electromagnetic radiation. Preferably, the source of electromagnetic radiation is configured to provide energy at or near the resonant wavelength of the agglomerated detector nanoparticles. In this way, release of the detectable signal means may only occur following both target-analyte induced agglomeration of the detector nanoparticles and the subsequent irradiation of these agglomerates with electromagnetic radiation.

In some cases, the source of electromagnetic radiation comprises a red laser diode. The red laser diode may deliver light at the resonant wavelength of the agglomerated detector nanoparticles, such as in the range 600 - 650 nm, e.g. 633 nm. In certain cases, the resonant wavelength may be away from the absorption maxima of Hb and $HbO_2$ present in blood (434 nm and 414 nm, respectively) and/or away from absorption wavelengths of the components of the device and/or away from absorption wavelength maxima of plasma. In some cases, the source of electromagnetic radiation is a light emitting diode (LED). In particular cases, this LED may be configured to provide energy at a wavelength of 415 - 425 nm.

In some cases in accordance with this and other aspects of the invention, the thermoresponsive polymer comprises at least one polymer selected from the group consisting of: poly(styrene sulfonate) (PSS), poly(diallyldimethylammonium chloride) (PDADMAC), poly(allylamine) (PAH), poly(N-isopropylacrylamide), poly[2-(dimethylamino)ethyl methacrylate] (pDMAEMA), hydroxypropylcellulose, poly(vinylcaprolactame) and polyvinyl methyl ether

In some cases in accordance with this and other aspects of the invention, the thermoresponsive polymer comprises alternating layers of poly(styrene sulfonate) (PSS) and poly(diallyldimethylammonium chloride) (PDADMAC) or of PSS and poly(allylamine) (PAH). This allows for the thickness of the polymer to be easily controlled, simply by varying the number of layers.

In some cases the thermoresponsive polymer comprises poly(allylamine) (PAH). In some cases the thermoresponsive polymer comprises poly(alpha)methylstyrene.

In some cases, the thermoresponsive polymer is between 60 - 100 nm thick. The present inventors have found that a polymer thickness in this range is preferred in order to provide sufficient rigidity to the polymer partition and to prevent release of the signal means before irradiation.

In some particularly preferred cases, the thermoresposive polymer comprises an odd number of layers. It is known that an odd number of layers will cause the thermoresponsive polymer to swell and break at temperatures above 60°C, due to repulsion between layers resulting in uncompensated charges. Accordingly, in some preferred cases, irradiation of the agglomerated detector nanoparticles produces a local temperature increase of at least 20°C, 40°C, or 60°C.

In some cases, the thermoresponsive polymer comprises a surface layer of poly(allylamine) (PAH).

In some preferred cases, detector nanoparticle agglomerates localise to the partition following agglomeration. In some cases this is via a specific binding pair, preferably the target analyte and the first and/or second probe of the detector nanoparticles. Accordingly, in some cases, the surface layer of the thermoresponsive polymer (e.g. a PAH layer) may be functionalised with the first and/or second probe of the detector nanoparticles. In this way, agglomerated detector nanoparticles can be localised to the partition using the same binding strategy which underlies detector nanoparticle agglomeration. The thermoresponsive polymer may have probe sequences (e.g. that specifically hybridise to the mutant sequence of interest) bound to or incorporated therein, e.g. bound to or incorporated into the surface layer of the thermoresponsive polymer. This arrangement helps to bring the agglomerated nanoparticles into close proximity to the thermoresponsive layer and thereby increases transfer of energy from irradiated nanoparticles to the thermoresponsive polymer (rather than for example the energy dissipating into the solution). However, it is specifically contemplated herein that in some cases there may be no binding probe present in or on the thermoresponsive polymer. The inventors have found that under certain conditions, for example depending on sedimentation rates of the nanoparticle agglomerates, the agglomerated nanoparticles come into close proximity to the thermoresponsive polymer without the need for a probe within or on the polymer.

In some cases, the first and second detector nanoparticles each comprise both said first and second probes. In particularly preferred cases, the first and second detector nanoparticles are the same.

In some cases the detector nanoparticles comprise a core of metal atoms, preferably a noble metal. In particular, the metal may be silver.

In some cases, the diameter of the detector nanoparticle core is in the range of 10 to 100 nm, such as in the range 40 to 70 nm.

In some cases, the signal means is selected from the group consisting of: streptavidin, an antibody, an enzyme (e.g. horse radish peroxidase), a fluorophore, a dye, one or more quantum dots, and one or more latex beads.

In some cases, the signal means is a plurality of signal nanoparticles. The present inventors have found that nanoparticles provide a useful advantage as a signal means, in that they allow use of a permeable or semi-permeable partition to close the signal reservoir. An air-tight seal, as would be required to prevent leakage of e.g. a liquid dye, is sensitive to differences in pressure; pressure-induced rupture or weakening of the partition material might therefore result in unwanted release of the signal means, and an increased incidence of false positive results. Advantageously, nanoparticles are small enough to be contained in a suitable signal reservoir, yet large enough that the partition closing the reservoir can be made permeable to allow equalisation of air pressure. In certain cases, the system may provide negative pressure in the device to facilitate flow. Therefore it is specifically contemplated herein that the partition closing the reservoir may not be permeable to air. Preferably, the number of signal nanoparticles released from the signal reservoir exceeds (preferably greatly exceeds) the number of detector nanoparticles required to effect said release. In certain cases the ratio of released signal means (e.g. signal nanoparticles) to detector nanoparticles is 1000:1 or more than 1000:1, for example 10^6:1 (i.e. a million-fold more signal means (e.g. signal nanoparticles) than detector nanoparticles or even higher). The large number of signal means (e.g. signal nanoparticles) relative to the number of detector nanoparticles helps to provide signal amplification, which in turn allows for more convenient detection, e.g., visible detection without the need for highly sensitive imaging apparatus.

In some cases, the signal nanoparticles comprise a core of metal atoms, preferably a noble metal. In particular, the metal may be gold. In some particularly preferred cases, the metal core of the signal nanoparticles and the metal core of the detector nanoparticles are different metals. This allows for detector nanoparticles to be used to direct a heat transfer to the thermoresponsive polymer by irradiation at a wavelength that will not be absorbed by the signal nanoparticles.

In some cases, the diameter of the signal nanoparticle core is in the range of 10 to 20 nm. In some cases, the signal nanoparticles have a spherical morphology. In other cases, the signal nanoparticles have a rod-shaped morphology. Other signal nanoparticle morphologies are also contemplated, and may be easily applied to the invention; for example, nanotubes, nanoboxes, and nanoclusters. The use of multiple different signal nanoparticle morphologies allows for different colour signals to be observed following signal nanoparticle release. This can be advantageous, e.g., in providing easily observable colour-coded outputs from the method.

In some cases, the signal nanoparticles are functionalised with a thiol terminated polyoxyethylene nonyl phenyl ether (IgeSH). It has been found that functionalisation with IgeSH stabilises nanoparticles against agglomeration, even following drying and redissolution.

In some cases, the signal reservoir comprises one or more of said microspheres, e.g., the thermoresponsive microspheres. The average diameter of the microspheres may be in the range 1 to 20 $\mu$m, such as in the range 5 to 10 $\mu$m.

In some cases, the signal reservoir comprises a plurality of said microspheres, and the surface layer of the shell is negatively charged or is positively charged.

In some cases, the signal reservoir comprises a plurality of said microspheres, and the microspheres are provided in a microchannel adjacent to and/or in contact with a plurality of packing particles of larger diameter than the microspheres. In particular, the packing particles may comprise glass, silica or agarose beads. The packing particles may have an average diameter in the range 20 to 200 $\mu$m, optionally in the range 50 to 150 $\mu$m.

In some cases, the signal reservoir comprises one or more cavities in a support material, preferably a macroporous silicon substrate. In some cases, the cavities are microcavities, microwells, or micropores. In some preferred cases, each cavity may contain detectable signal means at a concentration of at least $4 \times 10^5$ nanoparticles/$\mu$m$^3$, for example if nanoparticles are spherical with a radius of 7.5nm, and the cavities take the form of tubes of radius 0.5pm and depth 10$\mu$m and assuming relatively dense packing of the spherical nanoparticles. In this way, breakdown of the partition closing the signal reservoir by a potentially small number of agglomerated detector nanoparticles will result in the release of a comparatively large volume of signal means, thereby producing an amplification of the signal.

In some cases, release of the detectable signal means from the signal reservoir is observed as a colour signal. In some preferred cases, the release of the detectable signal means is observable with the naked eye. In some yet further preferred cases, the detectable signal means is captured for detection on a retention strip following release from the signal reservoir. In this way, visualisation of the release of the signal means from the signal reservoir is greatly facilitated. When the signal means is a plurality of signal nanoparticles, the capture of these on a retention strip allows their release to be easily observed as a colour change resulting from the distance-dependent optical properties of the nanoparticles.

In some cases in accordance with this aspect of the invention, the detector nanoparticle probes are oligonucleotides complementary to first and second regions of a target nucleic acid. In some cases, one or both oligonucleotide probes are covalently linked to the detector nanoparticle core via a linker group. In certain cases, the probe comprises modified or non-natural nucleotides. In particular, use of nucleotides that add rigidity to the oligonucleotide backbone, reducing the flexibility of natural DNA is contemplated herein. The more rigid oligonucleotides enhance probe-target specificity, which is desirable in particular when detecting a single base mutation against a background of wild-type DNA. In some cases the oligonucleotides may comprise 2'-o-methyl nucleotides. In some cases the oligonucleotides may comprise locked nucleic acids (LNAs).

In some particular cases, the linker group may comprise a thiol group. In some further cases, the linker group may comprise a C10 - C20 alkyl and/or glycol spacer. The incorporation of a spacer molecule facilitates binding of the probes to the target nucleic acid when attached to the detector nanoparticle core. In some cases the probes comprise or consist of a thiol bond to the nanoparticle core linked to a PEG spacer in turn linked to a C18 spacer in turn linked to the probe sequence itself. The spacer may limit or prevent the negatively charged probe from binding to the nanoparticle surface directly.

In some cases in accordance with this aspect of the invention, the first and second probe oligonucleotides bind regions of the target nucleic acid spaced 15 - 50 base pairs apart. This serves to favour binding of the target nucleic acid to discreet detector nanoparticles, as the short sequence difference structurally hinders binding to first and second probes on the same nanoparticle. In some particularly preferred cases, the probe oligonucleotides are: as set forth in Example 1, 5, 9 or 10.

In some cases, each detector nanoparticle has between 200 - 300 probe molecules.

In certain cases, the detectable signal means comprises a first member of a specific binding pair. In some cases, said first member is biotin or streptavidin. Accordingly, in some cases in accordance with this aspect of the invention, the signal nanoparticles comprise a first member of a specific binding pair and the retention strip comprises a corresponding second member of said specific binding pair. In some preferred cases, said first member and second members are selected from biotin and streptavidin. In some cases, the first member of the specific binding pair is covalently linked to the signal nanoparticle core via a linker group. In some further cases, this linker group comprises a thiol group, and optionally further comprises a C10 - C20 alkyl and/or glycol spacer.

In some cases, each detector nanoparticle has between 200 - 300 binding members.

In some cases in accordance with this aspect of the invention, the target nucleic acid has a sequence which is a variant of a wild-type sequence, and the first or second detector nanoparticle probes bind to the variant sequence in preference to the wild-type sequence. In some preferred cases, the variant sequence comprises a single nucleotide polymorphism (SNP), and the first or second detector nanoparticle probe hybridises to a portion of the target nucleic acid which comprises the SNP position. It was demonstrated that detector nanoparticles functionalised with first and second oligonucleotide probes can agglomerate in a specific manner, to distinguish between single-base differences in a target nucleic acid.

In some cases in accordance with this aspect of the invention, the nucleic acid is DNA. Accordingly, in some cases, the nucleic acid is a gene or fragment thereof suspected of being associated with a disease, while in some other cases the nucleic acid is a gene or fragment thereof known to be associated with a disease. In some particularly preferred cases, the nucleic acid is a gene or fragment thereof associated with cancer. In some other cases, the nucleic acid is RNA.

In some cases, the target nucleic acid comprises a mutation associated with cancer or other disorder having a genetic component. Examples of disorders having a genetic component and for which mutation detection in accordance with the present invention may be employed include: cystic fibrosis, haemochromatosis, phenylketonuria, Polycystic kidney disease, Sickle-cell disease, Spinal muscular atrophy, Tay-Sachs disease, Crohn's disease, and Huntington's disease. The mutation may be selected from the group consisting of: a single nucleotide change, a deletion, an insertion (including a trinucleotide repeat) or a sequence translocation.

In particular, the mutation may be in a gene selected from the group consisting of: human epidermal growth factor receptor (EGFR) of NCBI Gene ID: 1956; human Breast cancer 1 early onset (BRCA1) of NCBI Gene ID: 672; the human BRAF gene of NCBI Gene ID: 673; and the human KRAS proto-oncogene of NCBI Gene ID: 3845.

In certain cases, the mutation may be selected from the group consisting of:

the EGFR c.2573T>G (encoding L858R) Mutation;
the EGFR c.2369C>T (encoding T790M) Mutation;
an EGFR exon 19 deletion mutation; and
a KRAS mutation associated with colorectal cancer.

In some cases the test sample may be suspected of containing the target analyte, while in some other cases the test sample will be known to contain the target analyte. In some cases, the test sample may be an environmental sample, in particular a food or water sample. In some other cases the test sample may be a biological sample. In some particular cases, the test sample may be blood, blood plasma, urine, or other biofluids and their derivatives.

In some cases in accordance with this aspect of the invention, the method further comprises the step of heat denaturation of the test sample prior to contacting said test sample with the detector nanoparticles. In some further cases, the method may further comprise the step of providing a thermostabilising agent prior to heat denaturation of the test sample. In some cases, the thermostabilising agent is glucose. The provision of a thermostabilising agent can be useful in limiting protein denaturation in the test sample which might otherwise cause undesirable increases in sample viscosity. Alternatively or additionally, the method may comprise a step in which the sample is diluted and/or treated with an agent to remove albumin, such as Cibacron blue dye sepharose (for example Blue Sepharose 6 fast flow from GE Healthcare Life Sciences).

In some cases in accordance with this aspect of the invention, the method further comprises the step of providing a plurality of blocking probes, said blocking probes being capable of specific binding to the antisense strand of the target nucleic acid. In some cases the method further comprises the step of contacting an excess of said blocking probes (e.g. a 2- to 10-fold excess, such as a 5-to 7-fold excess or even a 6-fold excess, of blocking probes relative to the number of detection nanoparticle probes) with the test sample under conditions suitable for the binding of the blocking probes to the antisense strand of the target nucleic acid, thereby preventing the reannealing of sense and antisense strands of the target nucleic acid. In this way, the antisense strand of the target nucleic acid is removed from the system, allowing the test sample to be highly and specifically enriched for the sense strand of the target nucleic acid prior to contact with the detector nanoparticles.

In certain cases, the blocking probes comprise modified nucleotides, such as 2'-o-methyl nucleotides.

In some cases in accordance with this aspect of the invention, the method further comprises the step of providing a thermostabilising agent prior to heat denaturation of the test sample.

In certain cases in accordance with this aspect of the invention, the source of electromagnetic radiation is a laser diode or a light emitting diode, and irradiation of the agglomerated detector nanoparticles produces a local temperature increase of at least 20°C, 40°C, or 60°C. Preferably, the local temperature increase is at least 40°C.

In some cases, the surface of the thermoresponsive polymer layer is functionalised with the first and/or second probe of the detector nanoparticles, thereby causing detector nanoparticle agglomerates to localise to the thermoresponsive polymer layer.

In some cases, the detector nanoparticles comprise a core of silver atoms and the signal nanoparticles comprise a core of gold atoms.

In some cases, the signal nanoparticles comprise a first member of a biotin-streptavidin binding pair and the retention strip comprises a corresponding second member of said biotin-streptavidin binding pair.

In some cases in accordance with the invention, the blocking probe and detector nanoparticle probes are 2'-o-methyl oligonucleotides.

In a second aspect, the present invention provides a device for detecting the presence or absence of a target analyte in a test sample, comprising:

i) a detection compartment, containing a plurality of detector nanoparticles comprising a first detector nanoparticle functionalised with a first probe specific for a first region of a target analyte and a second detector nanoparticle functionalised with a second probe specific for a second region of said target analyte; and

ii) a signal amplification zone containing a detectable signal means contained in a signal reservoir by a partition that is selectively disruptable following target analyte-induced agglomeration of the detector nanoparticles, wherein the signal amplification zone further comprises an integrated source of electromagnetic radiation configured to provide energy at the resonant wavelength of the detector nanoparticles, and wherein the partition comprises a thermoresponsive polymer layer.

In some cases, the detection compartment further comprises an integrated source of electromagnetic radiation configured to provide energy at the resonant wavelength of the detector nanoparticles, and the partition separating the detection compartment and signal reservoir is a thermoresponsive polymer layer.

In some cases, the signal amplification zone further comprises an integrated source of electromagnetic radiation configured to provide energy at the resonant wavelength of the detector nanoparticles, and wherein the partition comprises a thermoresponsive polymer layer.

In some cases, the signal reservoir comprises one or more microspheres having a core comprising said signal means and a shell enclosing said core, and wherein the shell provides said partition.

In some preferred cases, the thermoresponsive polymer is as defined above in connection with the first aspect of the invention. In particular, the thermoresponsive polymer may comprise at least one polymer selected from the group consisting of: poly(styrene sulfonate) (PSS), poly(diallyldimethylammonium chloride) (PDADMAC), poly(allylamine) (PAH), poly(N-isopropylacrylamide), poly[2-(dimethylamino)ethyl methacrylate] (pDMAEMA), hydroxypropylcellulose, poly(vinylcaprolactame) and polyvinyl methyl ether

In some particularly preferred cases in accordance with this second aspect of the invention, the thermoresponsive polymer comprises an odd number of layers, as it is known that an odd number of layers will cause the thermoresponsive polymer to swell and break at temperatures above 60°C, due to repulsion between layers resulting in uncompensated charges.

In some cases in accordance with this second aspect of the invention, the source of electromagnetic radiation may be a laser diode or a light emitting diode (LED). The source of electromagnetic radiation may be adapted to produce light at a wavelength that corresponds to the resonant frequency of the detector nanoparticles, when in an agglomerated state. For example, the source of electromagnetic radiation may be a red laser diode having a wavelength in the range 600 to 650 nm (e.g. 633 nm). Alternatively, the source of electromagnetic radiation may be a LED preferably configured to provide energy at a wavelength of 415 - 425 nm.

In some cases, the detection compartment of the device may be functionalised with Optodex® (CSEM) to reduce protein interference with nanoparticle agglomeration.

In some cases, the partition separating the detection compartment and signal reservoir is functionalised with a member of a specific binding pair in order to localise detector nanoparticle agglomerates to the partition. In some particular cases, the partition is functionalised with the first and/or second probe of the detector nanoparticles. In this way, agglomerated detector nanoparticles can be made to localise to the partition using the same binding strategy underlying nanoparticle agglomeration. This dual function of the probes is advantageous in terms of design and manufacture of the device.

In some cases, in accordance with this second aspect of the invention, the thermoresponsive polymer comprises a surface layer of poly(allylamine) (PAH). Accordingly, in some cases the surface PAH layer is functionalised with the first and/or second probe of the detector nanoparticles.

In some cases in accordance with this and other aspects of the invention, the first and second detector nanoparticles each comprise both said first and second probes. In some particularly preferred cases, the first and second detector nanoparticles are the same.

In some cases, the detector nanoparticles comprise a core of metal atoms, preferably a noble metal. In particular cases the metal is silver. In some cases, the diameter of the detector nanoparticle core is in the range of 10 to 100 nm, e.g. in the range 40 to 70 nm.

In accordance with this second aspect of the invention, the signal means may be as defined in connection with the first aspect of the invention. In particular, the signal means may comprise streptavidin, an antibody, an enzyme (e.g. horse radish peroxidase), a fluorophore, a dye, one or more quantum dots, and/or one or more latex beads.

In some cases in accordance with this and other aspects of the invention, the signal means is a plurality of signal nanoparticles. The present inventors have found that nanoparticles provide a useful advantage as a signal means, in that they allow use of a permeable or semi-permeable partition to close the signal reservoir. An air-tight seal, as would be required to prevent leakage of e.g. a liquid dye, is sensitive to differences in pressure; pressure-induced rupture or weakening of the partition material might therefore result in unwanted release of the signal means, and an increased risk of false positive results. Advantageously, nanoparticles are small enough to be contained in a suitable signal reservoir, yet large enough that the partition closing the reservoir can be made permeable to allow equalisation of air pressure. Preferably, the number of signal nanoparticles released from the signal reservoir exceeds the number of detector nanoparticles required to bring about said release.

In some cases, the signal nanoparticles comprise a core of metal atoms, preferably a noble metal. In particular cases the metal is gold. In some cases, the diameter of the signal nanoparticle core is in the range of 10 to 20 nm.

In some particularly preferred cases, the metal core of the signal nanoparticles and the metal core of the detector nanoparticles are different metals. In this way, the detector nanoparticles can be used to direct a heat transfer to the thermoresponsive polymer by irradiation at a wavelength that is not absorbed by the signal nanoparticles or other components of the device.

In some cases, the signal nanoparticles may have a spherical morphology. In other cases, the signal nanoparticles may have a rod-shaped morphology. Other signal nanoparticle morphologies are also contemplated and may be easily applied to use in the device. Different nanoparticle morphologies allow for different colour signals to be observed following signal nanoparticle release, which may be advantageous in providing colour coded readouts from the device.

In some cases, the signal nanoparticles are functionalised with a thiol terminated polyoxyethylene nonyl phenyl ether (IgeSH). Functionalisation with IgeSH stabilises nanoparticles against agglomeration, even following drying and redissolution.

In some cases, the signal reservoir comprises one or more cavities in a support material, preferably a macroporous silicon substrate. The use of a macroporous silicon substrate allows for the preloading of signal means on a large scale; the substrate can then be cut to size as required, providing considerable advantages in terms of device manufacture.

In some cases, the cavities are microcavities, microwells, or micropores. In some preferred cases, each cavity may contain detectable signal means at a concentration of at least $4 \times 10^5$ nanoparticles/$\mu m^3$). In this way, breakdown of the partition closing the signal reservoir by a potentially small number of agglomerated detector nanoparticles will result in the release of a comparatively large volume of signal means, thereby producing an amplification of the signal. Preferably, breakdown of the partition closing multiple cavities is required to generate a detectable signal, so as to minimise false positives in cases of e.g. mechanical disruption of the partition opening a small number of cavities.

In some cases in accordance with the second aspect of the invention, the signal reservoir may comprise a plurality of microspheres as defined in connection with the first aspect of the invention. In particular, the microspheres may have an average diameter in the range 1 to 20 $\mu m$, such as in the range 5 to 10 $\mu m$.

In some cases, the signal reservoir comprises a plurality of said microspheres, and the surface layer of the shell is negatively charged or is positively charged.

In some cases, the signal reservoir comprises a plurality of said microspheres, and said microspheres are situated in a microchannel in the signal amplification zone and are adjacent to and/or in contact with a plurality of packing particles. The packing particles may be of larger diameter than the microspheres. In particular, the packing particles may comprise glass, silica or agarose beads. The packing particles may have an average diameter in the range 20 to 200 $\mu m$, such as in the range 50 to 150 $\mu m$.

In some cases, the microchannel in which the microspheres are housed together with the packing particles forms a microfluidics retaining chamber.

In some cases in accordance with this second aspect of the invention, the device further comprises a signal display region, e.g. which may be in communication with the detection compartment. In some preferred cases, this signal display region comprises a retention means to capture the detectable signal means following its release from the signal reservoir. In particular cases, the retention means is a retention strip. The incorporation of the signal display region in the device allows for convenient on-device readout.

In some cases in accordance with this and other aspects of the invention, the detector nanoparticle probes are oligonucleotides complementary to first and second regions of a target nucleic acid. In some cases, one or both oligonucleotide probes are covalently linked to the detector nanoparticle core via a linker group. In some particular cases, the linker group may comprise a thiol group. In some further cases, the linker group may comprise a C10 - C20 alkyl and/or glycol spacer.

In some cases in accordance with this aspect of the invention, the first and second probe oligonucleotides bind regions of the target nucleic acid spaced 15 - 50 base pairs apart.

In some cases, each detector nanoparticle has between 200 - 300 probe molecules. In certain cases, the probe oligonucleotides are 2'-o-methyl oligonucleotides.

In some cases, the detector nanoparticle probe oligonucleotides bind a gene associated with a disease. In some preferred cases, the gene is a gene associated with cancer.

In some cases, the first or second probe oligonucleotides are complementary to a target nucleic acid sequence which is a variant of a wild-type sequence. In particular cases, this variant sequence comprises a single nucleotide polymorphism (SNP) and the first or second probe oligonucleotide hybridises to a portion of the target nucleic acid comprising said SNP position. In some case, the variant sequence comprises a mutation associated with cancer or other disorder having a genetic component. Examples of disorders having a genetic component and for which an associated mutation may be employed as the target analyte in accordance with the present invention include: cystic fibrosis, haemochromatosis, phenylketonuria, Polycystic kidney disease, Sickle-cell disease, Spinal muscular atrophy, Tay-Sachs disease, Crohn's disease, and Huntington's disease. The mutation may be selected from the group consisting of: a single nucleotide change, a deletion, an insertion (including a trinucleotide repeat) or a sequence translocation.

In some case, the mutation is in a gene selected from the group consisting of: human epidermal growth factor receptor (EGFR) of NCBI Gene ID: 1956; human Breast cancer 1 early onset (BRCA1) of NCBI Gene ID: 672; the human BRAF gene of NCBI Gene ID: 673; and the human KRAS proto-oncogene of NCBI Gene ID: 3845.

In particular, the mutation may be selected from the group consisting of:

the EGFR c.2573T>G (encoding L858R) Mutation;
the EGFR c.2369C>T (encoding T790M) Mutation;
an EGFR exon 19 deletion mutation; and
a KRAS mutation associated with colorectal cancer.

In some cases in accordance with this and other aspects of the invention, the detectable signal means comprises a first member of a specific binding pair. In some preferred cases, said first member is biotin or streptavidin. Accordingly, in some particularly preferred cases, the signal nanoparticles comprise a first member of a specific binding pair and the retention strip of the signal display region comprises a corresponding second member of said specific binding pair, wherein said first member and said second member are selected from biotin and streptavidin. In this way, signal nanoparticles can be captured onto the retention strip following their release, and are easily observed as a visible colour change at the strip resulting from distance-dependent optical properties of the nanoparticles.

In some cases, the first member of the specific binding pair is covalently linked to the signal nanoparticle core via a linker group. In some particular cases, the linker group may comprise a thiol group. In some further cases, the linker group may comprise a C10 - C20 alkyl and/or glycol spacer. In some cases each detector nanoparticle has between 200 - 300 binding members.

In some cases in accordance with this second aspect of the invention, the device further comprises a sample inlet in communication with the detection compartment.

In some cases, the device further comprises a lysis compartment disposed between said sample inlet and detection compartment, said lysis compartment comprising a heating element. Preferably, the heating element is a thin film heating element as these are more rapid and efficient than bulk heating components, and can be easily powered by an on-device power source (e.g. a button battery).

In certain cases, the sample inlet may comprise a blood separation means. Preferably, this will be a self-powered integrated microfluidic blood analysis system (SIMBAS), which is more efficient and cost-effective than other membrane-based blood separation technologies. In other cases, the sample inlet contains a thermostabilising agent, such as glucose or Cibacron blue.

In some cases the device of this and other aspects of the present invention may be a device for detection of, e.g., circulating DNA that may take the form of a stand-alone, self-powered micro fluidic system. The device may comprise and/or be made of polymeric materials. The device may be designed to enable a sequential work flow. In particular, the following work-flow stages, and corresponding device features, may be provided: on-sample preparation (plasma extraction and target DNA enrichment), sample incubation with detection nanoparticles, detection signalling particles release and final binding assay on a lateral flow assay strip to give a naked eye detectable signal. The device may, in some cases, hold a sample volume of less than 500 microliters. The microfluidic system may, in some cases, take the form of a hybrid device integrating paper-like microfluidics into a plastic cassette. Sample transport inside the device may, in some cases, be driven by negative pressure (e.g. customized poly(dimethylsiloxane) ("PDMS") micropumps).

In some cases in accordance with this second aspect of the invention, the detection compartment further contains a plurality of blocking probes specific for the antisense strand of a target nucleic acid. The blocking probes may be as defined in connection with the first aspect of the invention.

In some cases in accordance with this and other aspects of the invention, the blocking probes are 2'-o-methyl oligonucleotides.

In a third aspect, the present invention provides a device for detecting the presence or absence of a target analyte in two or more test samples, comprising the elements of the second aspect of the invention in parallel paths on the same device.

In a further aspect, the present invention provides a device of the second or third aspects of the invention for use in a method of diagnosis or prognosis of a mammalian subject, wherein the test sample is a biological sample that has been obtained from said subject, or a method of detection of a bacterial, parasitic, or other biological contaminant, wherein the test sample is an environmental sample.

In a further aspect, the present invention provides a kit comprising:

i) a device having:

a sample inlet;

a lysis compartment comprising a heating element;

an integrated source of electromagnetic radiation;

a plurality of thermoresponsive microspheres having a core comprising signal means and a shell enclosing the core, wherein the shell comprises a thermoresponsive polymer; and

a signal display region comprising a retention strip to capture the signal means following release from the microspheres;

optionally,

ii) one or more populations of a blocking probe capable of specific binding to the antisense strand of a target nucleic acid; and

iii) one or more populations of a plurality of detector nanoparticles functionalised with a first probe specific for a first region of the sense strand of said target nucleic acid and a second probe specific for a second region of said target nucleic acid.

The present invention includes the combination of the aspects and preferred features described except where such a combination is clearly impermissible or is stated to be expressly avoided. These and further aspects and embodiments of the invention are described in further detail below and with reference to the accompanying examples and figures.

## Brief Description of the figures

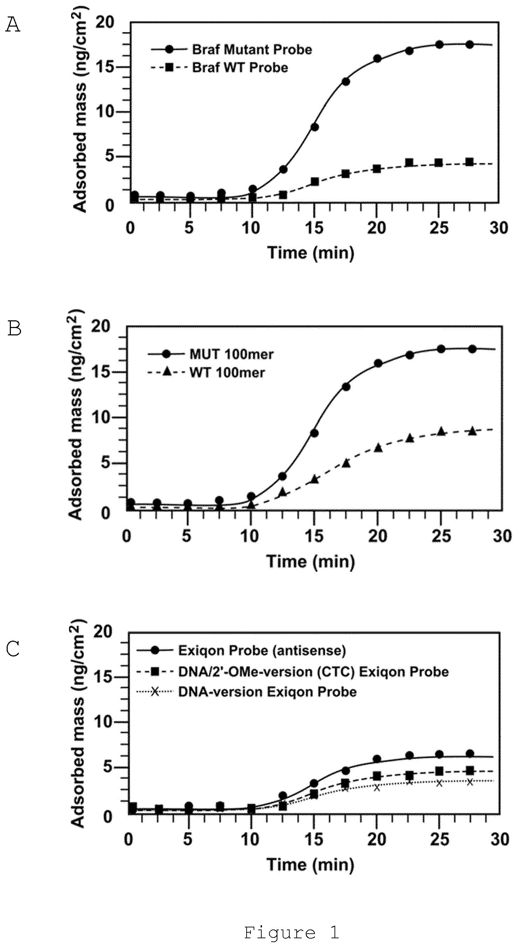

**Figure 1** shows the binding of designed oligonucleotide probes to (synthetic) mutant and wild-type BRAF gene sequences, as assessed by surface plasmon resonance. **A:** shows the degree of binding of mutant (circles) and wild-type (squares) probes to the mutant BRAF gene sequence (500 nM of synthetic ssDNA 100-mer); the mutant probe binds specifically to the mutant BRAF gene, while the wild-type probe binds comparatively weakly. **B:** shows preferential binding of the designed mutant probe to the mutant BRAF gene sequence (500 nM of synthetic ssDNA 100-mer; circles) as compared with the wild-type BRAF gene sequence (500 nM of synthetic ssDNA 100-mer; triangles). **C:** shows the relative strength of binding of mutant probe to mutant BRAF gene for three different probe chemistries. Comparison of the binding curves determined the dissociation constants (KD) for the different probe chemistries as: DNA = 2.41 nM; 2'-O-Me = 1.81 nM; LNA = 7.55 nM.

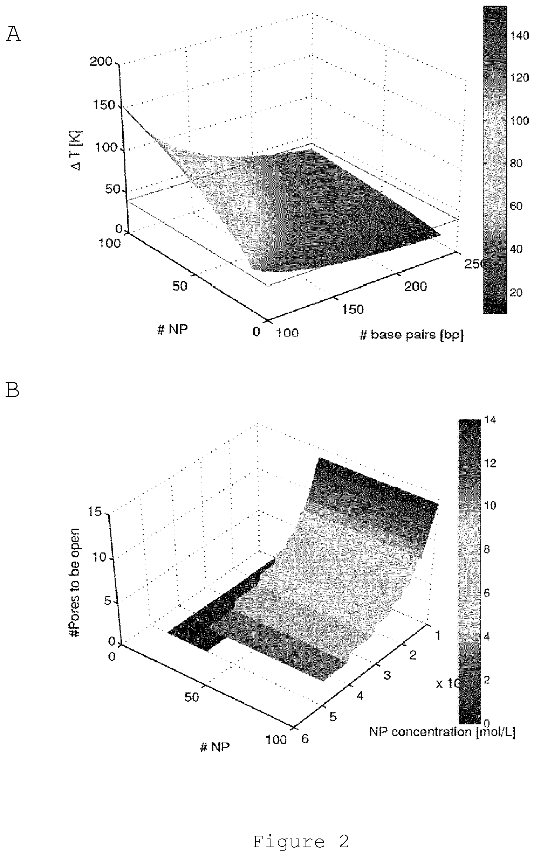

**Figure 2** shows computational modelling data from *in silico* simulation of the INDICATE device, used to optimise certain aspects of the device prior to manufacture. **A:** shows temperature change ($\Delta$T) as a function of the distance between detector nanoparticles against the number of agglomerated nanoparticles when illuminated uniformly with a 100 mW LED laser (intensity of $1 \times 10^7$ W/m$^2$). From this it was determined that an agglomerate of 50 detector nanoparticles, in which the distance between nanoparticles is >50 nm (i.e. >15 base pairs) will elicit a temperature increase of more than 40°C when illuminated uniformly with a 100mW LED laser. **B:** shows the number of micropores required to open to produce a detectable signal, as a function of both the concentration of signal nanoparticles in the micropores and the number of agglomerated detector nanoparticles. On the basis that each micropore can contain between 0.2 - 1 $\times 10^9$ signal nanoparticles, it was determined that at least 8 micropores would need to be opened to result in a visible signal ($8 \times 10^9$ signal nanoparticles).

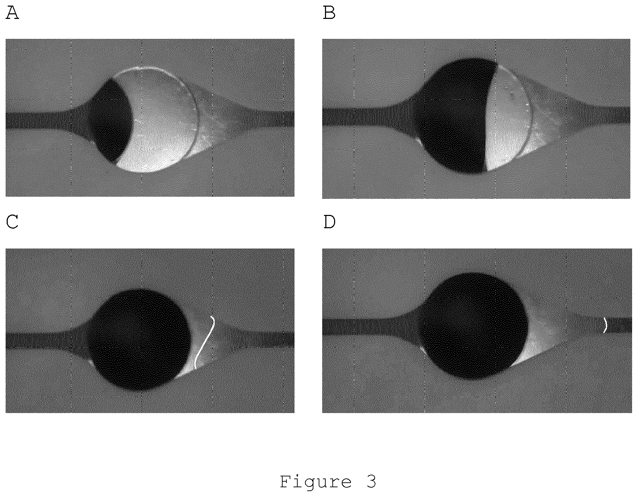

**Figure 3** shows on-device separation of a whole blood sample to give plasma by the self-powered integrated microfluidic blood analysis system (SIMBAS). Separation of clear blood plasma from whole blood is shown with increasing time (**A, B, C,** and **D**). Clear blood plasma at the outlet of the trench structure is indicated with a white line to guide the eye.

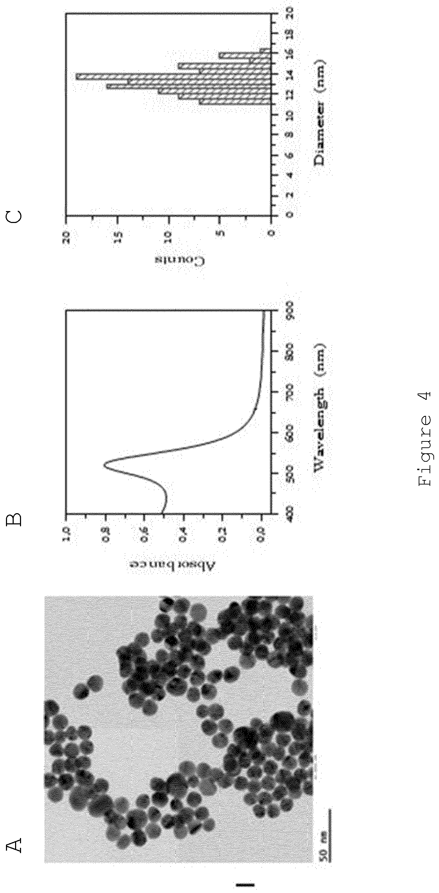

**Figure 4** shows the characterisation of gold nanoparticles synthesised by the Turkevich method. **A:** shows a transmission electron microscopy (TEM) image of the gold nanoparticles with 50 nm scale indicated. **B:** shows UV-Vis spectroscopic characterisation of the gold nanoparticles, with a localised surface plasmon band at 519 nm. **C:** shows the size distribution histogram of synthesised nanoparticles. A mean diameter of 13.3 $\pm$ 1.2 nm was determined by analysing a sample of over 100 nanoparticles.

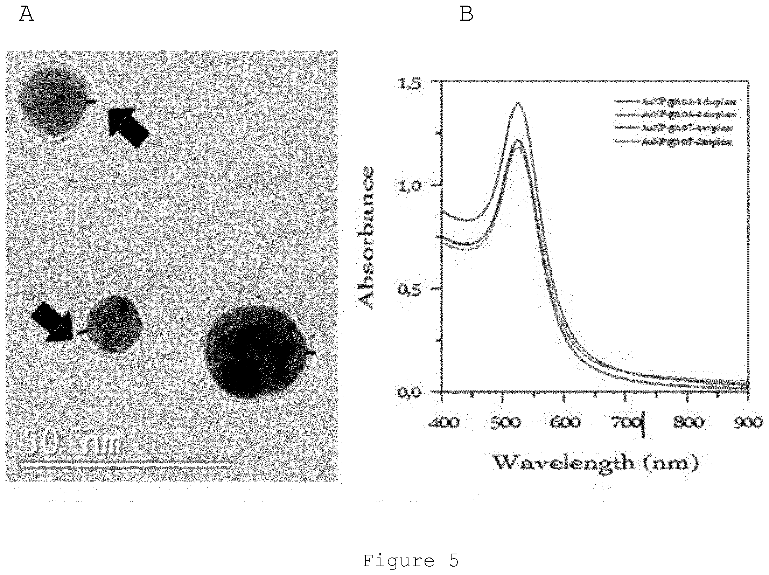

**Figure 5** shows the characterisation of gold nanoparticles following functionalization with DNA probes. **A:** shows a TEM image of the DNA-functionalised nanoparticles, in which the bound DNA can be observed as a faint molecular shell surrounding the nanoparticles (approximate thickness 1.4 nm). **B:** shows UV-Vis spectroscopic characterisation of the functionalised nanoparticles, in which a plasmon band red-shift of around 3 nm is observed in all samples after DNA binding; indicative of the formation of a molecular shell as observed in the TEM images.

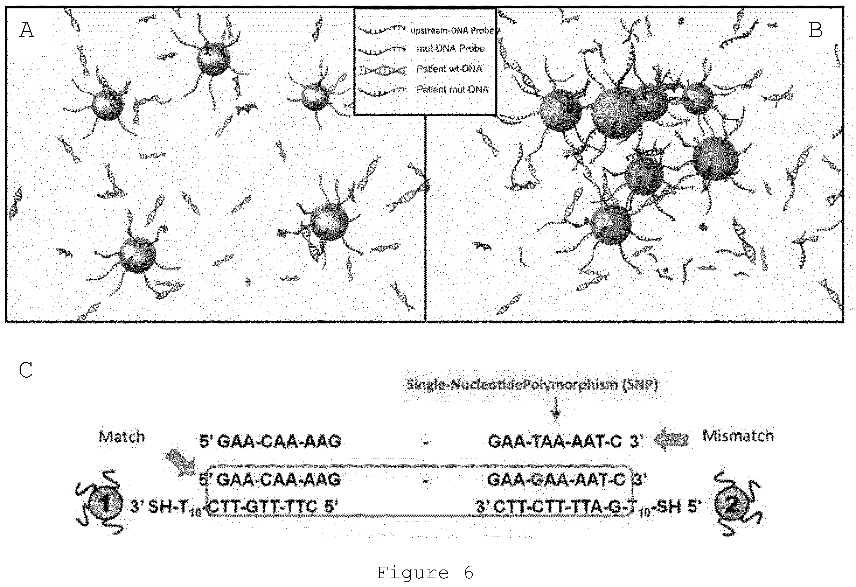

**Figure 6** shows a schematic representation of the "sandwich-like" binding of probe sequences required for the agglomeration of nanoparticles in the presence of the target nucleic acid. **A:** the wild-type sequence of the target gene can bind the upstream probe, but does not bind the probe specific for the mutant gene, and therefore does not result in nanoparticle agglomeration (i.e. it remains a monodispersal). **B:** the mutated sequence of the target gene can bind both the probe specific for the mutant gene and the upstream probe. The target sequence acts as a molecular bridge, binding the two probes on separate nanoparticles thereby causing nanoparticle agglomeration. **C:** Mismatch between the wild-type sequence of the target gene and the probe specific for the mutant gene prevents "sandwich-like" binding.

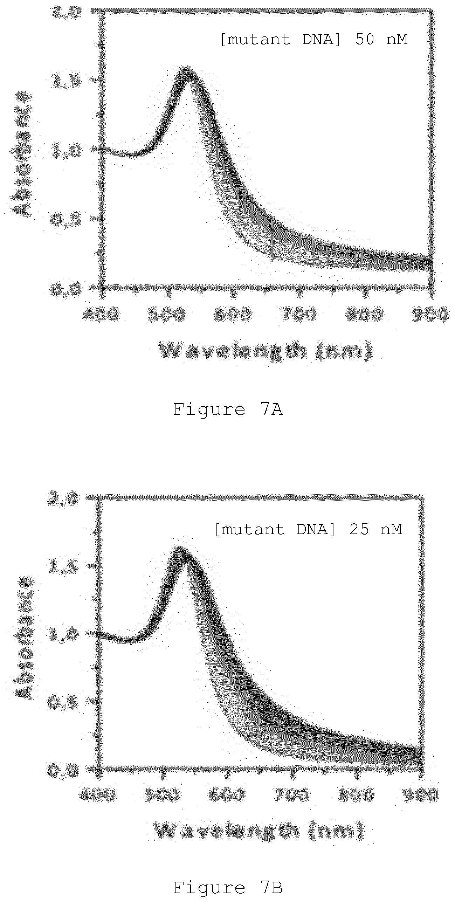

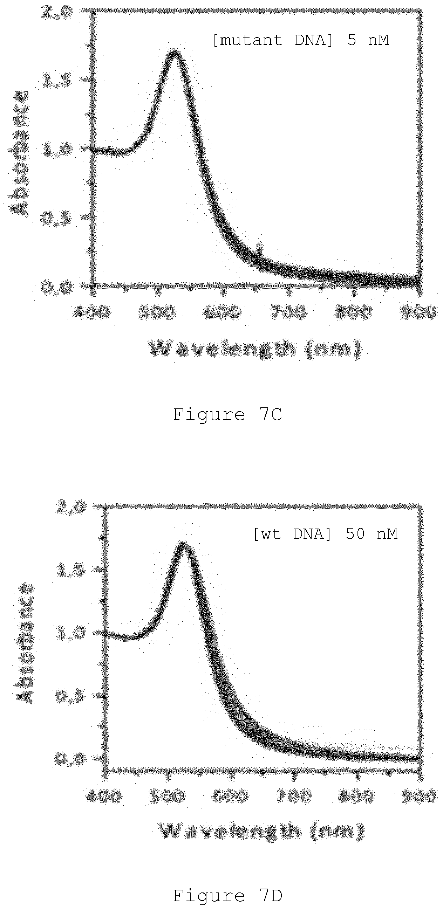

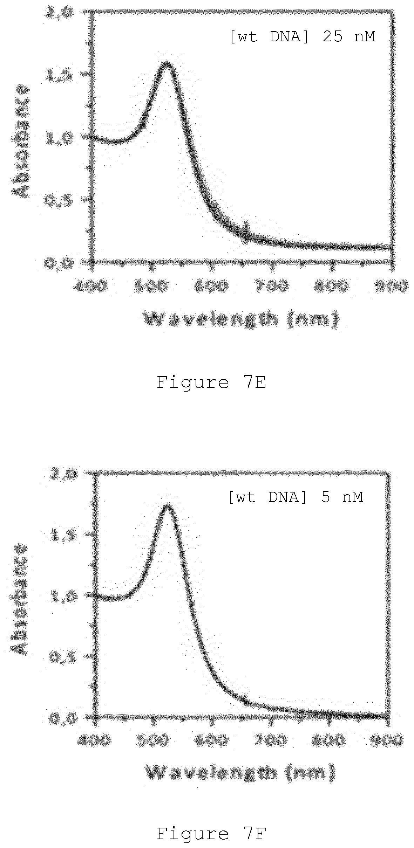

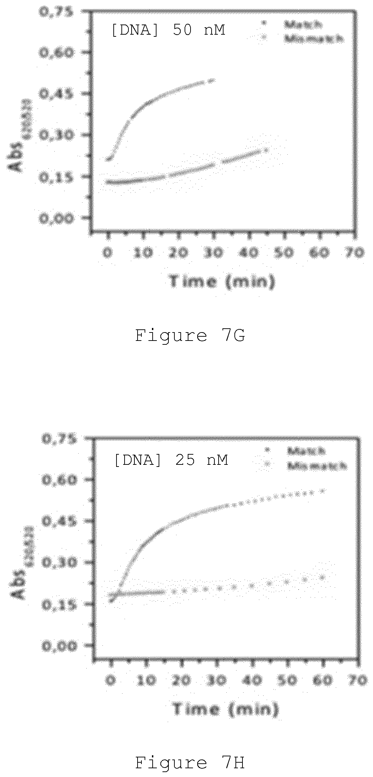

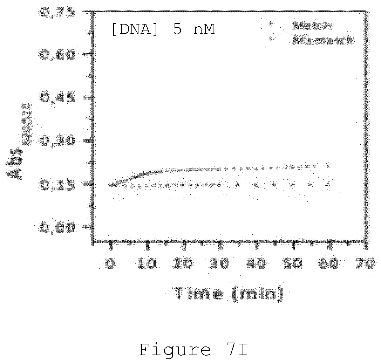

**Figure 7** shows the effect of analyte (target DNA) concentration on nanoparticle agglomeration for a given concentration of nanoparticle bound probes (1.5 nM), as determined by UV-Vis spectroscopy. **A - C:** show shifts in the UV-Vis spectra with time for varying concentrations (50, 25, and 5 nM, respectively) of (matched) mutant DNA. **D - F:** show shifts in the UV-Vis spectra with time for varying concentrations (50, 25, and 5 nM, respectively) of (mismatched) wild-type DNA. A single base-pair mismatch in the target DNA results in less pronounced agglomeration of nanoparticles, indicating that nanoparticles functionalised with DNA probes can agglomerate in a manner that can distinguish between single-base differences in the target sequence. **G - I:** show comparative kinetic data for different concentrations of matched (i.e. mutant) and mismatched (i.e. wild-type) target DNA.

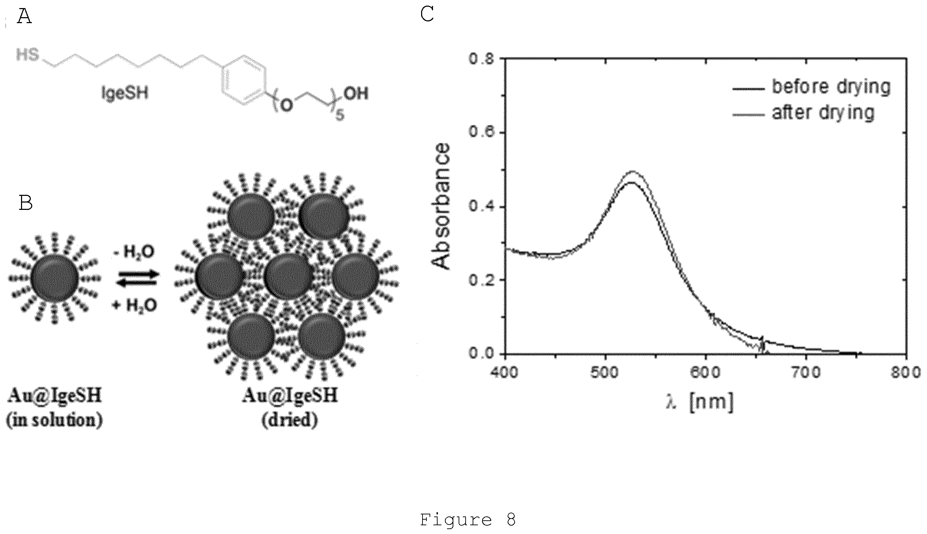

**Figure 8** shows the characterisation of gold signal nanoparticles synthesised according to the Turkevich method. **A:** shows the chemical structure of the thiol terminated polyoxyethyelene nonyl phenyl ether (IgeSH) with which signal nanoparticles were functionalised to stabilise them against agglomeration. **B:** shows a schematic representation of the reversible drying/hydratation process of IgeSH functionalised signal nanoparticles. **C:** shows UV-Vis spectra of the IgeSH functionalised signal nanoparticles in water before and after drying.

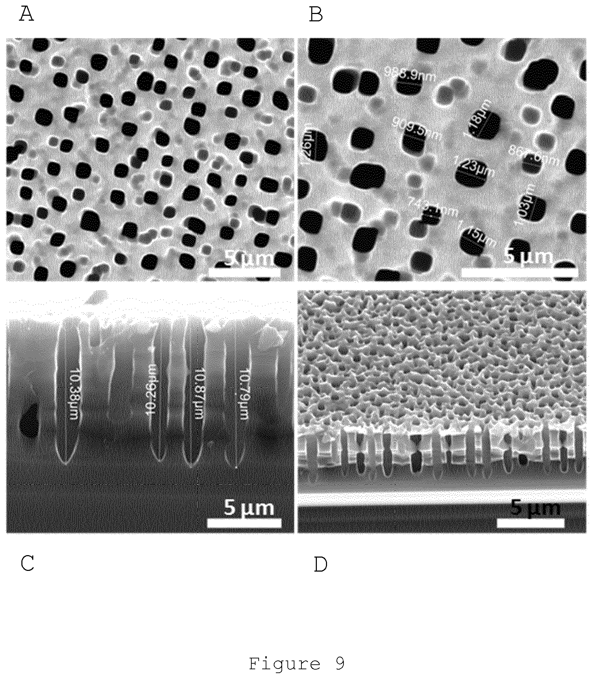

**Figure 9 A** - **D:** show scanning electron microscopy (SEM) images of the macroporous silicon substrate used to form the signal reservoir from a variety of angles and magnifications. Approximate dimensions of the micropillar cavities have been indicated alongside a 5 $\mu$m scale bar.

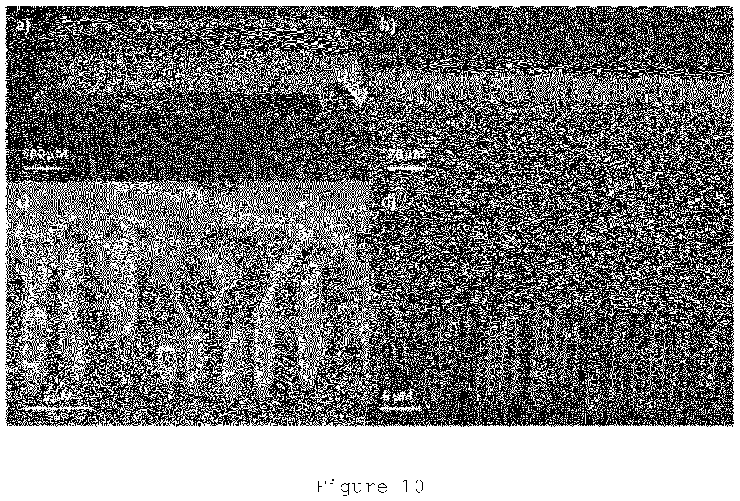

**Figure 10 A** - **D:** show SEM images of the signal reservoir substrate loaded with signal nanoparticles at various magnifications. 500, 20, and 5$\mu$m scale bars are indicated. Gold signal nanoparticles are observed as areas of white colour.

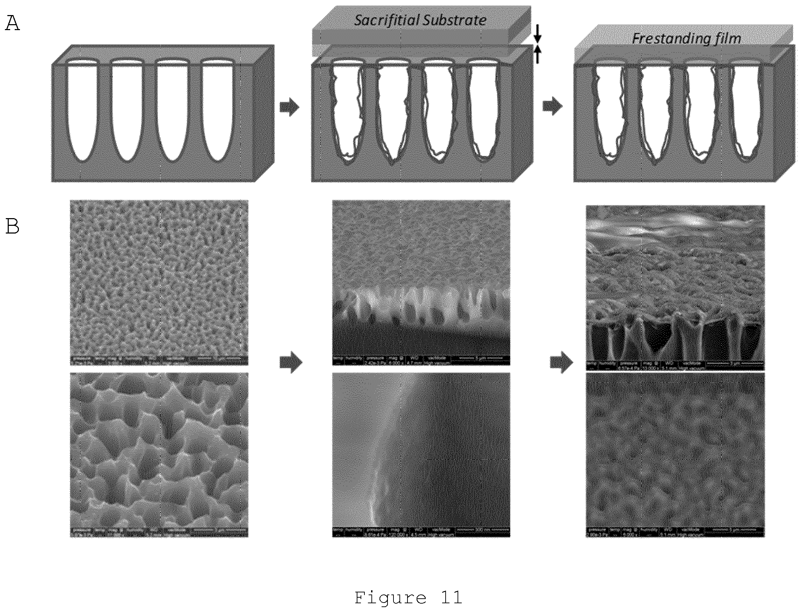

**Figure 11** shows the process of layer-by-layer transfer of a thermoresponsive polymer membrane onto the surface of the macroporous silicon substrate. **A:** shows a schematic representation of this process: first, the silicon substrate is coated with film 1 (PEI(PSS/PAH)$_{10}$PSS), while film 2 (PEI (PSS/PDADMAC)$_{12}$) is formed on a sacrificial substrate of poly(methylmethacrylate) (PMMA); both films are then attached through electrostatic interaction and pressure; finally, the sacrificial substrate is removed by dissolution or by mechanically peeling it off, to arrive at the final film. **B:** shows SEM images taken during each step of this process, with the thickness of the final film measured at around 60-100 nm.

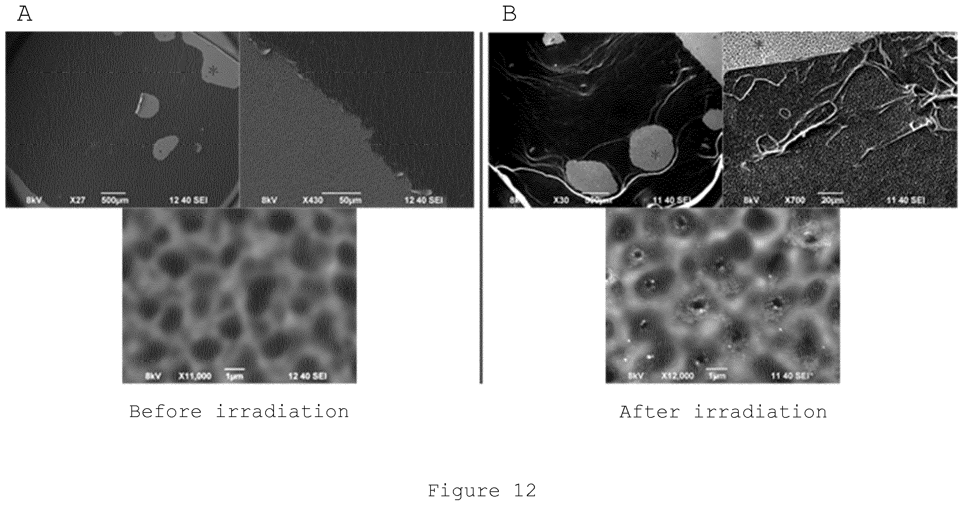

**Figure 12** shows SEM images of the thermoresponsive film doped with gold nanorods before (**A**) and after (**B**) irradiation with a 980 nm laser (0.3 W/cm$^2$). Ruptures in the film are clearly visible following laser irradiation.

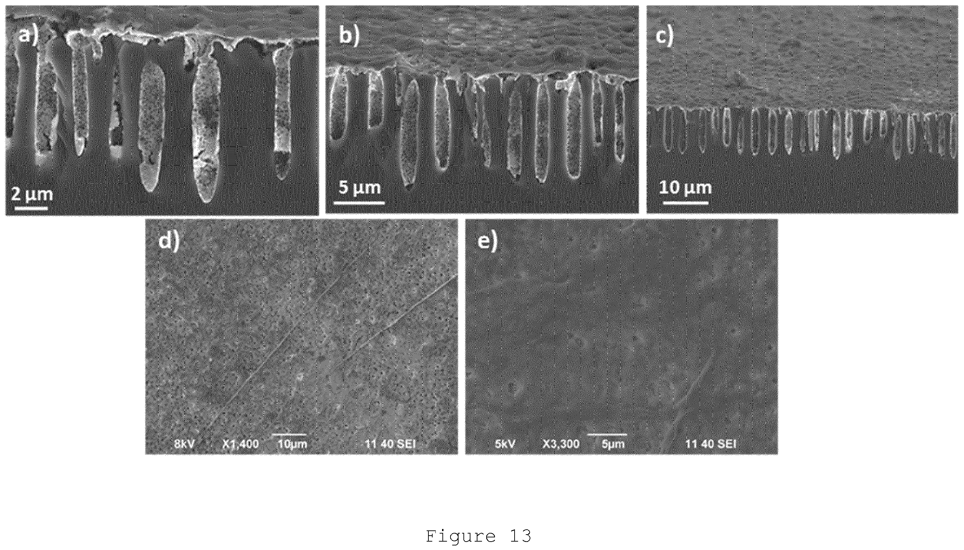

**Figure 13 A** - **E:** show SEM images of the signal reservoir substrate loaded with signal nanoparticles in the micropillar cavities, and with the thermoresponsive film 2 present on the surface. 2, 5, and 10 $\mu$m scale bars are indicated. Ruptures in the film are visible following laser illumination (**D & E**).

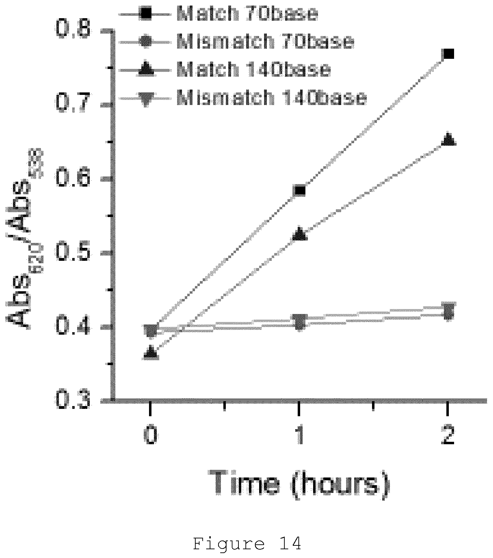

**Figure 14** shows a plot of absorbance ratio vs. time for 70-base match probe (squares); 70-base mismatch probe (circles); 140-base match probe (upright triangles); and 140-base mismatch probe (upside down triangles) . A clear time-dependent increase in Abs$_{620}$/Abs$_{538}$ ratio is observed for both match probes, which significantly surpasses the near base-line, minimal absorbance change seen for the mismatch probes. The results indicate target-specific nanoparticle agglomeration in which a single nucleotide difference has been discriminated.

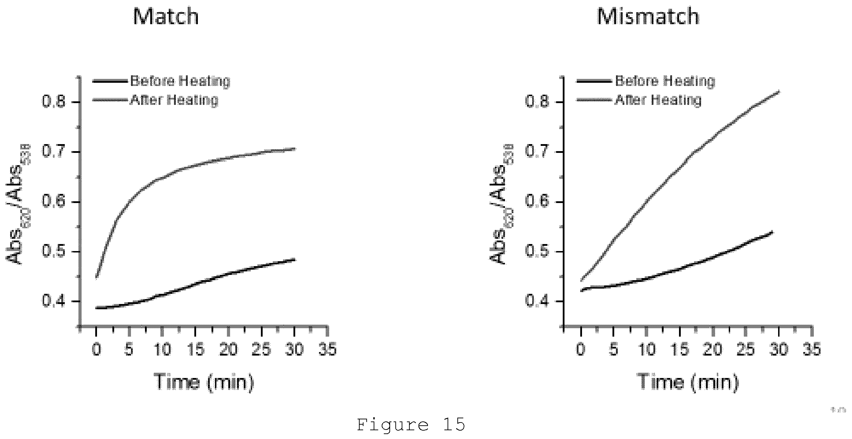

**Figure 15** shows (left-hand panel) a plot of Abs$_{620}$/Abs$_{538}$ ratio against time in minutes for match probe before heating (lower curve) and after heating (upper curve); and (right-hand panel) a plot of Abs$_{620}$/Abs$_{538}$ ratio against time in minutes for mismatch probe before heating (lower curve) and after heating (upper curve).

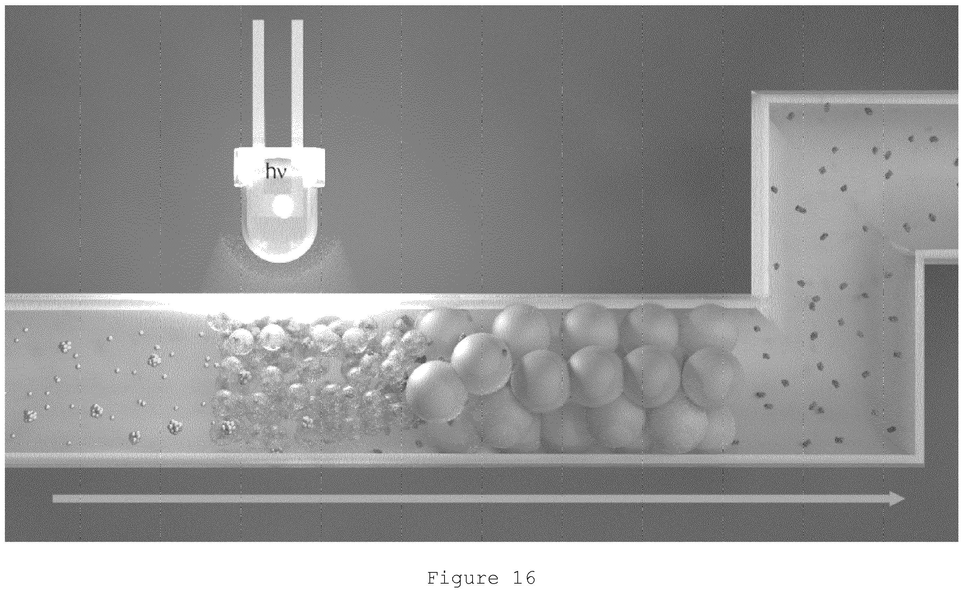

**Figure 16** shows a schematic diagram of the signal amplification zone (retention chamber) in which agglomerated detector nanoparticles are shown entering from the left and encountering a plurality of microspheres loaded with signal means (shown below a laser light source "hv" represented by a light bulb). The agglomerated nanoparticles come into close association with the microspheres. To the right of the microspheres a plurality of silica or glass beads are shown in closely-packed formation, having larger diameter than the microspheres. The beads act to retain the intact microspheres. Absorption of laser light by the agglomerated nanoparticles causes plasmon resonance and heating of the nanoparticles, which in turn causes disruption of the thermosensitive microsphere shells, allowing the signal means to escape. The signal means passes through the beads to the right in the direction of the arrow, the flow being facilitated by a micropump (not shown).

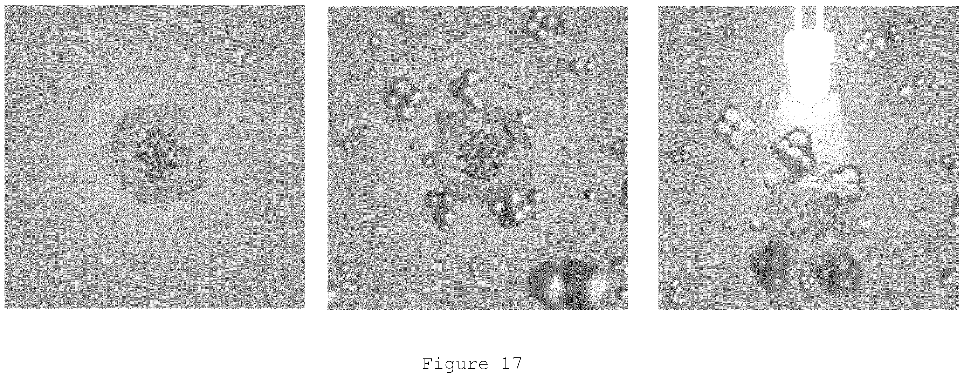

**Figure 17** shows a schematic representation of the agglomerated nanoparticle-triggered release of signal means from a microsphere. The left-hand panel depicts a microsphere having a shell of a thermoresponsive polymer and a core containing signal means (e.g. readily detectable molecules). The middle panel depicts the microsphere now surrounded with agglomerated nanoparticles many of which are in close contact with the surface of the microsphere shell. The right-hand panel depicts the microsphere and agglomerated nanoparticles now illuminated by a light source (e.g. a laser diode having a wavelength corresponding to the resonant frequency of the agglomerated nanoparticles). The agglomerated nanoparticles are heated by the light source and the heat is transferred to the shell of the microsphere causing it to breakdown, thereby resulting in release of the signal means from the core of the microsphere.

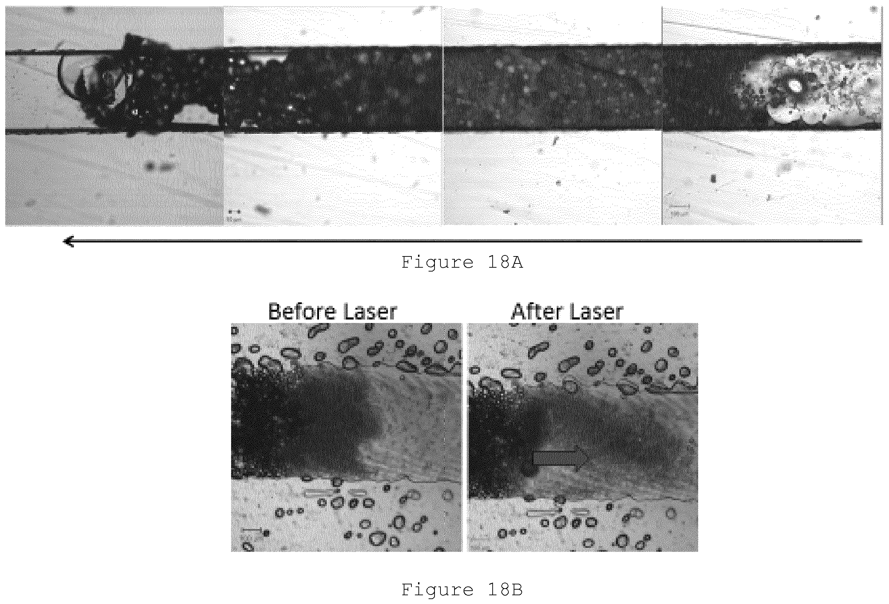

**Figure 18** shows **A**) particle packing within cyclic olefin polymer (COP) channels in which glass beads of diameter 75 $\mu$m clogged the channel thereby retaining dextran-FITC-loaded microspheres; and **B**) release of dextran-FITC from microspheres (compare before laser on the left with after laser on the right.

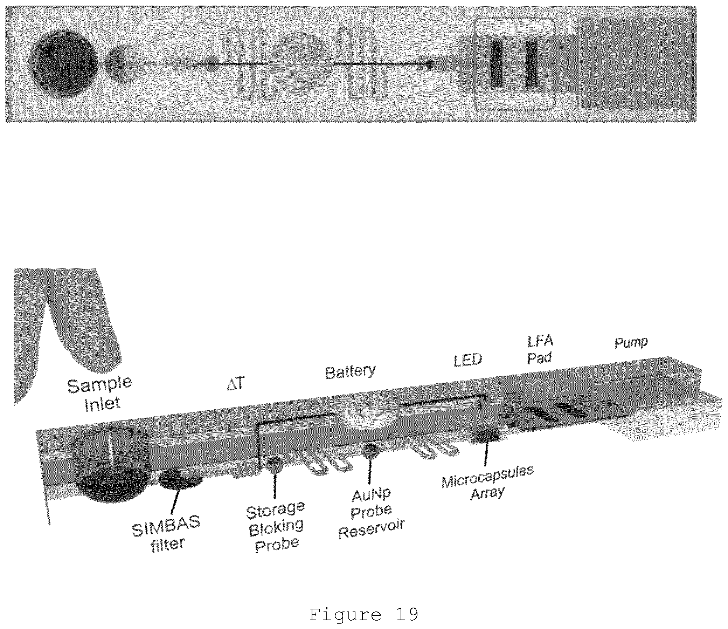

**Figure 19** shows a schematic diagram of an integrated device of the invention illustrating the relationship between the different modules. The upper panel shows a top view of the integrated device. The lower panel shows the device in perspective view. The sample inlet is shown in the left. Moving right, the SIMBAS filter, heater ("ΔT"), blocking probe storage area, battery, detector nanoparticle ("AuNP") probe reservoir, light emitting diode (LED), microcapsules array, lateral flow assay (LFA) pad and pump, are shown.

## Detailed description of the invention

In describing the present invention, the following terms will be employed, and are intended to be defined as indicated below.

Nanoparticles

As used herein, "nanoparticle" refers to a particle having a nanomeric scale, and is not intended to convey any specific shape limitation. In particular, "nanoparticle" encompasses nanospheres, nanotubes, nanoboxes, nanoclusters, nanorods and the like. In certain embodiments the nanoparticles and/or nanoparticle cores contemplated herein have a generally polyhedral or spherical geometry. References to "diameter" of a nanoparticle or a nanoparticle core are generally taken to mean the longest dimension of the nanoparticle or nanoparticle core, respectively. For nanoparticles having a substantially polyhedral or spherical geometry, the shortest dimension across the particle will typically be within 50% of the longest dimension across the particle and may be, e.g., within 25% or 10%.

As used herein, "corona" refers to a layer or coating, which may partially or completely cover the exposed surface of the nanoparticle core. The corona includes a plurality of ligands covalently attached to the core of the nanoparticle. Thus, the corona may be considered to be an organic layer that surrounds or partially surrounds the metallic core. In certain embodiments the corona provides and/or participates in passivating the core of the nanoparticle. Thus, in certain cases the corona may include a sufficiently complete coating layer substantially to stabilise the core. In certain cases the corona facilitates solubility, such as water solubility, of the nanoparticles.

Nanoparticles are small particles, e.g. clusters of metal or semiconductor atoms, that can be used as a substrate for immobilising ligands.

As used herein, the term "detector nanoparticle" refers to a nanoparticle that is functionalised with one or more probes specific for a target analyte of interest. Preferably, the nanoparticles have cores having mean diameters between 10 and 100 nm, more preferably between 20 and 60 nm. When the ligands are considered in addition to the cores, preferably the overall mean diameter of the particles is between 22 and 70 nm, more preferably between 30 and 60 nm and most preferably between 40 and 50 nm. The mean diameter can be measured using techniques well known in the art such as transmission electron microscopy.

As used herein, the term "signal nanoparticle" refers to a nanoparticle that is intended to function as a signal molecule. This signal may arise as a result of intrinsic properties of the nanoparticle e.g. the distance-dependent optical properties of noble metal nanoparticles, or via incorporation of a detectable label. Preferably, the nanoparticles have cores having mean diameters between 5 and 30 nm, more preferably around 15 nm. When the ligands are considered in addition to the cores, preferably the overall mean diameter of the particles is between 7 and 40 nm, more preferably between 10 and 30 nm and most preferably between 15 and 20 nm. The mean diameter can be measured using techniques well known in the art such as transmission electron microscopy.

The core material can be a metal or semiconductor and may be formed of more than one type of atom. Nanoparticle cores may also be formed from alloys. Preferably, the core material is a metal selected from Au or Ag. The cores of the nanoparticles preferably comprise at least 500 atoms (e.g. gold atoms) to provide core diameters in the nanometre range.

In some embodiments, the nanoparticle or its ligand may comprise a detectable label. The label may be an element of the core of the nanoparticle or the ligand. The label may be detectable because of an intrinsic property of that element of the nanoparticle or by being linked, conjugated or associated with a further moiety that is detectable. Preferred examples of labels include a label which is a fluorescent group or a dye. Fluorescent groups include fluorescein, rhodamine or tetramethyl rhodamine, Texas-Red, Cy3, Cy5, etc., and may be detected by excitation of the fluorescent label and detection of the emitted light using Raman scattering spectroscopy (Y.C. Cao, R. Jin, C. A. Mirkin, Science 2002, 297: 1536-1539).

Agglomeration

As used herein, "agglomeration" refers to the formation of agglomerates of detector nanoparticles. "Agglomerates" are an assembly of detector nanoparticles joined by physical interaction between nanoparticle probes and the target analyte of interest. As the target analyte is the physical "bridge" between discreet detector nanoparticles, agglomeration may only occur when the target analyte is present. Agglomerates are not fixed units, and may change in size and shape; for example, large agglomerates may break down into smaller agglomerates, or small agglomerates may coalesce to form larger agglomerates.

*Thermoresponsive polymer*

As used herein, "thermoresponsive polymer" or "temperature-responsive polymer" refers to a polymer or combination of polymers that exhibit a drastic and discontinuous change of their physical properties with temperature. In particular, the thermoresponsive polymer may be a polymer that exhibits a change in solubility in water or aqueous solution with temperature. Specific examples include: poly(styrene sulfonate) (PSS), poly(diallyldimethylammonium chloride) (PDADMAC), poly(allylamine) (PAH), poly(N-isopropylacrylamide), poly[2-(dimethylamino)ethyl methacrylate] (pDMAEMA), hydroxypropylcellulose, poly(vinylcaprolactame) and polyvinyl methyl ether.

The following is presented by way of example and is not to be construed as a limitation to the scope of the claims.

## Examples

### Example 1 - Development of blocking and detection probes for BRAF mutation detection.

Mutations in the BRAF gene are found in more than 45% of melanomas, with up to 80% of these being the single base mutation T1799A (V600E). The detection of the status of this BRAF mutation is believed to be a reliable substitute for tumour testing.

In order to detect BRAF mutation status, two probes were developed:

1) A blocking probe, complementary to the antisense strand of the mutated DNA sequence of interest; this probe binds preferentially to the antisense strand of the mutated DNA sequence after heat denaturation of the double stranded DNA (dsDNA).

The blocking probe is short and is provided in excess, such that binding kinetics greatly favour the formation of blocking probe:antisense strand rather than the re-annealing of the mutated sequence dsDNA. This results in enrichment of the sense strand of the mutated DNA sequence for subsequent detection by the device.

2) A detection probe, complementary to the sense strand of the mutated DNA sequence and which forms the basis of the agglomeration event. This probe binds selectively to the mutated DNA sequence but not to the wild-type DNA sequence.

Mutated and wild-type BRAF sequences were synthesised, and their binding with detection probes for wild-type and mutant BRAF assessed by surface plasmon resonance (SPR) in PBS buffer. The mutated BRAF sequence was found to bind preferentially to the mutant BRAF probe (Figure 1A) rather than the wild-type probe. Mutant BRAF probe: 5'-TGG TCT AGC TAC AGA GAA ATC TCG -3' (SEQ ID NO: 1); WT BRAF probe: 5'-TGG ATC CAG ACA ACT GTT CAA ACT -3' (SEQ ID NO: 2) (corresponds to first 24 nucleotides of the synthetic sequence). Similarly, the mutant BRAF probe binds much more strongly to the mutated BRAF sequence than the wild type BRAF sequence (Figure 1B). These data demonstrate that the designed probes can bind specifically to mutated sequences of interest.

A number of different probe chemistries (DNA, 2'-o-Me, and LNA) were also tested (Figure 1C), in order to determine the relative strength of binding of mutant probe to mutant BRAF gene for each. DNA Exiqon probe: 5'-AT CGA GAT TTC TCT GTA GCT AG-3' (SEQ ID NO: 3); 2'-o-methyl probe: 5'-AT CGA GAT TTC TCT GTA GCT AG-3 (SEQ ID NO: 4) (both bind antisense strand of DNA mutated 70mer); LNA mutant probe: 5'-TG GTC TAG CTA CAG AGA AAT CTC G -3' (SEQ ID NO: 5) (binds sense strand of DNA mutated 70-mer). Comparison of the binding curves determined the dissociation constants (KD) for the different probe chemistries as: DNA = 2.41 nM; 2'-o-Me = 1.81 nM; LNA = 7.55 nM. From these data, the 2'-o-Methyl probe chemistry was determined to be most preferred.

### Example 2 - In silico modelling of INDICATE device characteristics.

In order to optimise the functional characteristics of the device, computational modelling was used to simulate the INDICATE device *in silico.*

First, individual mathematical equations responsible for each of the individual components were defined, and then combined using genetic algorithms (GA) (Goldberg and Deb, 1991, Foundations of Genetic Algorithms In Foundations of Genetic Algorithms, G. J. E. Rawlins, Ed. Morgan Kaufmann, San Mateo, CA). Such techniques are already well-established in optimising the design of plasmonic devices (Sukharev and Seideman, Nano Lett, 2006, Vol. 6, No. 4, pp 715-719).

Simulations were used to optimise the device in respect of: 1) high sensitivity (i.e. a low false negative rate); 2) high signal/noise ratio; and 3) high amplification rate of the input signal. Analysis was performed using multiphysics approaches within Matlab numerical computing (MathWorks) and Comsol Multiphysics (Comsol AB) software.

The separation of plasma from red cells in the sample module was modelled using probabilistic approaches for the filtration process (Roussel et al., Phys Rev Lett, 2007, Vol. 98, pp 114502). Electrothermal melting of the anneling and re-anneling of DNA in the lysis module was simulated using the two-state model of thermodynamics (Owczarzy et al., Biopolymers, 1997, Vol. 44, No. 3, pp 217-239). The binding energy associated with mutated DNA binding to probes in the signal amplification zone was modelled using the Berg von Hippel method (Berg and von Hippel, J Mol Biol, 1987, Vol. 193 pp 723-750), while the energy transfer between the laser and the agglomerated detector nanoparticles to the thermoresponsive polymer was modelled by classical heat transfer theory (Baffou and Quidant, Laser Photonics Rev, 2013 Vol 7, pp 171-187; Hohenester and Trügler, Comput Phys Commun, 2012, Vol. 183, pp 370-381).

To provide an initial performance range for the device, the size of the agglomerate formed when the mutated sense strand DNA is bound to the detector nanoparticles was modelled as follows:

$$S = \mathrm{N_{pNP}}\pi \left( \varPhi_{AgNP} + \frac{\mathrm{p}}{2} \right)^2$$

where S represents the surface covered by the agglomerate containing a number of DNA bound probe nanoparticles ($\mathrm{N_{pNP}}$), $\varPhi_{AgNP}$ represents the diameter of the silver nanoparticles, and p is the interparticle distance.

This was combined with a model of uniform illumination, based on previous theoretical and experimental work (Baffou et al., Appl Phys Lett, 2009, Vol. 94 pp 153109-153103):

$$\varDelta\mathrm{T} \approx \frac{\sigma_{\mathrm{abs}}\mathrm{I}}{\kappa} \frac{\ln(1+\sqrt{2})}{\pi} \frac{\mathrm{s}}{\mathrm{p}^2}$$

where $\sigma_{\mathrm{abs}}$, I, $\kappa$, S, and p are the absorption coefficient of the probe NPs, the power intensity provided by the LED source, the thermal conductivity of the agglomerate, the lateral size of the agglomerate and the interparticle distance, respectively.

Figure 2A shows $\Delta$T as a function of the distance between detector nanoparticles (in number of base pairs) against the number of agglomerated probe particles ($\mathrm{N_{pNP}}$) when illuminated uniformly with a 100mW LED laser (intensity of $1 \times 10^7$ W/m$^2$). From this analysis it was determined that an agglomerate of 50NPs, in which $d_{pNP-pNP}$ is >50 nm (i.e >15 base pairs), will result in a temperature increase of more than 40°C above room temperature.

By modelling the cylindrical micropores with fixed dimensions ($10\mu$m diameter by $500\mu$m), for a concentration range of signal nanoparticles of $10^{-5}$ - $5 \times 10^{-5}$ mol/L, we can profile the minimum number of micropores that are required to open against the number of probe nanoparticles contained in agglomerates. Figure 2B shows the number of micropores required to open as a function of both the concentration of signal nanoparticles in the micropores and the number of agglomerated detector nanoparticles. It should be noted that in the case of microwells of macroporous silicon substrate, the cavities have a diameter of approximately 1 $\mu$m and a depth of approximately 10 $\mu$m. In this case, applying the above approach to calculating the number of pores open for a positive signal gives a figure of 7 (nanoparticle concentration 0.1mol/L, number of nanoparticles per pore $4\times10^9$). Therefore, the above figures, while illustrative of certain embodiments are not limiting on the invention as defined further herein.

On the basis that each micropore can contain between 0.2 - $1 \times 10^9$ signal nanoparticles, it was determined that at least 8 micropores would need to be triggered to result in a visible signal ($8 \times 10^9$ signal nanoparticles). This threshold of visible signal helps prevents false positives on the device in case a single pore opens. However, as seen above, for cavities of dimensions 1 $\mu$m by 10 $\mu$m, the corresponding threshold may be calculated to be 7 pores open.

For this to occur, at least 50 probe nanoparticles per agglomerate per micropore are required. This corresponds to 400 copies of mutated sense strand DNA in total for a visible signal. Assuming a device efficiency of 25% gives a theoretical limit of detection (LOD) of 1,600 mutated DNA copies or 16,000 copies per ml of blood. This is well within the reported levels of mutated KRAS DNA copies or mutated BRAF copies in the blood of cancer patients (reported as between 50 - 180,000; Spindler et al., Clin Cancer Res, 2012, Vol. 18, pp 1177-1185).

Modelling of this type can also be used to tailor manufacture of the device in order to meet specific detection or market requirements. For example, while a device for diagnostic purposes requires high sensitivity, one that facilitates clinical decision making on the administration of expensive targeted therapies may benefit from high specificity (i.e. low rate of false positives).

### Example 3 - Validation of microfluidic blood separation.

Filtration of whole blood sample to give plasma is by the self-powered integrated microfluidic blood analysis system (SIMBAS) (Dimov et al., Lab Chip, 2011, Vol. 11, pp 845-850), which is more efficient and cost-effective than other membrane based blood separation technologies.

The SIMBAS system was adapted to accept higher volumes (100µl), through modification of the design. The principal changes to the original design (i.e. that described in the above Dimov et al., 2011 reference) were as follows.

A different fabrication technique was used to manufacture the chips. In this case, a quick and simple multi plastic thin films lamination (PMMA, PSA, COP) technique was employed.

The dimensions of the pool were scaled to a 60 microliters volume in order to obtain 40 microliters of plasma from a 100 microliters whole blood sample.

The channel aspect ratios were adapted to the manufacturing process restrictions.

The final channel dimensions were designed to reduce the hydraulic resistance of the chip.

A particular improvement was provided in the form of an absence of sharp edges in the side walls which were changed to blended edges. All the edges in the path proposed have been blended in order to avoid platelet aggregation and formation of clots which could easily block the flow.

Finally, the segments of the channel located at the inlet and outlet of the pool have been modified with a diffuser & nozzle shape, respectively. On the one hand, the diffuser slows down the flow when it is approximating to the edge of the pool to obtain a smooth fill process (homogeneity in filling the pool). On the other hand, the nozzle accelerates the plasma at the exit of the pool improving the flow of separated plasma and in fact the separation efficiency.

Figure 3 shows the separation of clear blood plasma from a whole blood sample with increasing time, demonstrating capability for on-device separation of blood.

### Example 4 - Detector nanoparticle synthesis and functionalization.

Gold nanoparticles (AuNPs) were synthesized according to the Turkevich method (Turkevich et al., Discuss Faraday Soc, 1951, Vol. 11, pp 55-75), to produce particles with a mean diameter of 13.3 $\pm$ 1.2 nm (estimated by analysing over 100 nanoparticles). UV-Vis characterization showed the localized surface plasmon band located at 519 nm (Figure 4). Gold detector nanoparticles were used during prototype development of the device, for their stable surface chemistry. In the final device, silver detector nanoparticles will be used.

Citrate-stabilized AuNPs were functionalized with single stranded DNA (ssDNA) according to recent protocols (Mirkin et al., Anal Chem, 2006, Vol. 7, pp 8313-8318). AuNPs stabilized with DNA exhibit colloidal stability in an aqueous solution containing anionic surfactant - sodium dodecyl sulphate (SDS), 0.01 wt%, as confirmed by UV-Vis spectroscopy. The plasmon band redshifts ∼3 nm in all samples after DNA binding, suggesting the formation of a molecular shell around the nanoparticles surface. TEM analysis confirms the formation of a molecular shell of a thickness of ∼1.4 nm (Figure 5).

### Example 5 - Validation of detector nanoparticle agglomeration in the presence of mutated target DNA.

Figure 6 shows a schematic representation of the "sandwich" binding of two probes spaced 15 - 50bp apart, used to interrogate varying concentrations of a target DNA analyte. When both probes are conjugated to detector nanoparticles they will hybridise with their respective complementary regions of the mutated sense strand DNA to form a bridge between discreet nanoparticles, thereby inducing agglomeration of free-floating detector nanoparticles.

Agglomeration was measured by colour shift and ratio of Abs (620/520) with time (Figure 7). Hybridisation using single-base mismatched DNA (Figure 7D - 7F) produced less pronounced aggregation and longer hybridization times as compared to the fully complementary sequence (Figure 7A - 7C), demonstrating that nanoparticles functionalised with oligonucleotide probes can agglomerate in a specific manner to distinguish between single-base mutations. Figure 7G - 7I show comparative kinetics for different concentrations of matched (i.e. mutant) and mismatched (i.e. wild-type) target DNA, in which clear differences exist between the kinetic profile of matched and mismatched sequences.

Two types of AuNPs conjugated with probes termed "1triplex" (HS-3'-Tio-CTT GTT TTC-5') (SEQ ID NO: 6) and "2triplex" (HS-5'-$T_{10}$-GAT TTT CTT C-3') (SEQ ID NO: 7) were prepared and used as stock solutions. The two complementary particles were mixed in a buffer containing 10 mM PBS, pH

7.4, 0.137 M NaCl and 2.7 mM KCl, and extra NaCl (0.2 M). We used three different concentrations of match and mismatch DNA, namely 5, 25 and 50 nM, giving in total 6 experiments. Regardless to the analyte concentration, the DNA with a single-based mismatch showed less pronounced aggregation and longer hybridization time, as compared to matching sequence.

Decreasing DNA analyte concentration by half, from 50 to 25 nM, increases the hybridization time from 9 to 12 minutes. The relatively large values of Abs620/520 ratio (0.4), at Th, for both concentrations of matching sequences, suggest a good performance of the sensor. Further decrease of the analyte concentration down to 5 nM has little effect on the Th ,but small value of Abs620/520 = 0.18, at Th, suggest underperformance in the particular conditions of this example. Therefore, the plasmonic sensor was able to differentiate single-base polymorphism at the analyte concentration of 25 nM in less than 15 minutes.

With the aim of further improving the detection limit of the matching sequence, we evaluated the performance varying the salt concentration. Two types of AuNPs conjugated with 1triplex and 2triplex were prepared and used as stock solutions. The two complementary particles were mixed in a buffer containing 10 mM PBS, pH 7.4, and 50 nM of matching and mismatching sequences. We used three different concentrations of NaCl, namely 0.2, 0.3 and 0.4 M, giving in total 6 experiments. With the increase of a concentration of NaCl the hybridization time decreases. High concentration of NaCl (0.4M) indeed makes shorter the Th (6 minutes), but the specificity of the sensor is affected since the hybridization of the mismatch sequence becomes significant. Note we want to keep the largest possible difference between match and mismatch at shortest possible hybridization time. Therefore, the upper limit of the salt concentration under the conditions of this particular example was found to be 0.3 M.

Conclusions and outlook:
The plasmonic sensor is capable of differentiating a single-base mutation at the concentration of 25 nM or higher, within less than 15 minutes.

The optimal salt concentration in detection experiments was found to be around 0.2 M.

The optimal particles concentration [NPs] in detection experiments was found to be 1.5 nM.

Further evaluation of the detection of longer ssDNA molecules (100 and 250 mer) using the sandwich system is contemplated.

Use of particles with larger absorption coefficient (nanorods) that allow the improvement of performance in term of sensing time (hybridization time) and sensitivity (limit of detection) is contemplated.

### Example 6 - Signal nanoparticle synthesis and functionalization.

Gold signal nanoparticles were synthesized according to the Turkevich method (Turkevich et al., Discuss Faraday Soc, 1951, Vol. 11, pp 55-75), and functionalised with a thiol terminated polyoxyethyelene nonyl phenyl ether (IgeSH). This molecule is amphiphilic and makes nanoparticles stable against agglomeration.

These signal NPs can be dried and redissolved in water without significant agglomeration. Figure 8 shows the chemical structure of the thiol terminated polyoxyethyelene nonyl phenyl ether (IgeSH), a schematic representation of the reversible drying/hydratation process of IgeSH functionalised signal nanoparticles, and the UV-Vis spectra of the IgeSH functionalised signal nanoparticles in water before and after drying (see Mao et al., Anal. Chem., 2009, Vol. 81, pp. 1660-1668).

The gold nanoparticles can be functionalized with a second ligand composed by a linear chains of poly (ethylene glycol) modified with a thiol group at one end and a biotin molecule at the other (see Li et al., J Phys Chem B., 2006, Vol. 110(32), pp. 15755-15762) . Thus, the thiol group will strongly bind to the gold surface, enabling double ligand functionalization. The biotin molecules will be used for immobilization on the retention strip which will be functionalised with streptavidin. In order to stabilize the nanoparticles and avoid non-specific interactions, an appropriate ratio of the two ligands IgeSH and biotin is required.

### Example 7 - Loading of signal nanoparticles in signal reservoir and synthesis of the thermoresponsive polymer membrane.

A macroporous silicon substrate was selected for use as the signal reservoir; these are fabricated by electrochemical etching of p-type silicon wafers. Figure 9 shows scanning electron microscopy (SEM) images of the macroporous silicon substrate with approximate dimensions of the micropillars indicated. These hollow micropillars have a volume of approximately 8 $\mu m^3$ (r= 0.5 $\mu m$, h= 10 $\mu m$).

Signal nanoparticles were loaded into the substrate micropillars from above, to form a signal reservoir. Figure 10 shows SEM images of the reservoir substrate loaded with signal nanoparticles at various magnifications; gold signal nanoparticles are observed as areas of white.

The silicon substrates are oxidized to have $SiO_2$ on the surface. It is therefore possible to wrap the surface with positively charged polymers. A layer-by-layer approach was used to develop the thermoresponsive film from alternating layers of poly(styrene sulfonate) (PSS) and poly(diallyldimethylammonium chloride) (PDADMAC). These polyelectrolytes self-assemble due to electrostatic interactions and are known to swell and break above 60 °C if the number of layers in the film is odd, due to repulsion between layers resulting in uncompensated charges.

A schematic representation of the layer-by-layer transfer of a film onto macroporous silicon substrate by removal of a sacrificial substrate is shown in Figure 11A. First, the silicon substrate was coated with film 1 (PEI(PSS/PAH)$_{10}$ PSS), while film 2 (PEI (PSS/PDADMAC)$_{12}$) was formed on a sacrificial substrate of poly(methylmethacrylate) (PMMA). Both films were then attached through electrostatic interactions and pressure. Finally, the sacrificial substrate was removed, either by dissolution with an organic solvent ($CH_2Cl_2$) or by mechanically peeling it off.

Figure 11B shows SEM images taken during the three steps of deposition of film 1, adhesion of film 2, and removal of the PMMA substrate. Films produced in this way can be formed at different thicknesses simply by controlling the number of layers and the ionic strength of the polyelectrolyte solution, etc. The thickness of the film measured from these images is around 60-100 nm.

*Example 8 - Validation of polymer breakdown by irradiation.*

In order to test the responsiveness of the film to heat/light, signal nanoparticles were deposited in the cavities of a macroporous Si substrate and the cavities covered with a (PSS/PDADMAC)$_{12}$ film doped with gold nanorods.

This was then illuminated with a 980 nm laser (0.3 w/cm$^2$), resulting in the formation of holes in the films. Figure 12 shows a (PSS/PDADMAC)$_{12}$ film doped with gold nanorods before and after laser illumination, with irradiation-induced ruptures in the film clearly visible. Similarly, Figure 13 shows SEM images of the Si substrate with signal nanoparticles accumulated within the pores and thermoresponsive film 2 on the surface. Ruptures in the film are visible following laser illumination (**D** & **E**).

*Example 9 - Detection probes and SNP discrimination*

**Functionalization of AuNP with EGFR Sequences**

In this study we focused on EGFR mutation that relates to NSCLC. To detect mutated analyte target, AuNP with diameter of 63 nm were functionalized with corresponding thiol-terminated-ssDNA (capture probes). Two batches of AuNPs were prepared; stabilized with MUT and WT capture probes that comprised following sequences: EGFR Capture Probe 5' (WT): 5'-A AAA TCT GTG 10T-SH-3' (SEQ ID NO: 8)
EGFR Capture Probe 3' (MUT): 5'-SH-10T GTT TGG C***CCG***CC C-3' (SEQ ID NO: 9)
To increase the affinity of the Capture Probe to the target, we introduced a ***2'-OMe*** modification in 3 bases in MUT capture probe. The AuNPs stabilised with capture probes, either WT and MUT, are colloidally stable over extended period of time in buffer solution (results not shown).

**Sensitivity & Selectivity Study - 23baseAnalyte**

Analyte sequences with a length of 23 bases were used for the assay: Match 23base: 5'-CAC AGA TTT TGG GC***G*** GGC CAA AC-3' (SEQ ID NO: 10) Mismatch 23base: 5'-CAC AGA TTT TGG GC***T*** GGC CAA AC-3' (SEQ ID NO: 11)

The sensitivity and selectivity of the assay was performed toward single-base mutation using Match and Mismatch. Two types of NP were mixed in PBS with and extra of 0.2 M NaCl in a UV micro cuvette. The concentration of [Au°] was 0.1 mM in all the experiments. Then, an aliquot of analyte was added to the solution. The aggregation of the particles was followed by UV-Vis spectroscopy. The aggregation degree was calculated as a ratio of absorbance at 620 and 538 nm. The concentration range for the analyte sequences was between 50 and 0.05 nM.

Although we could detect the match and mismatch down to 50 pM, no selectivity between the Match and Mismatch was observed.
To confirm UV-Vis results, Dynamic Light Scattering measurements were also carried out to know the size of the aggregates.

The size of the initial NPs was 86.3 and 72.9 nm for AuNP@MUT and AuNP@WT, respectively. After 30 minutes of aggregation, the size of the agglomerates was 250 nm approximately, for the match and the mismatch sequences.

**Sensitivity & Selectivity Study - 70-base& 140-base Analyte**

In parallel to the above study we checked the assay performance toward the detection of long sequences of DNA, 70 bases. Match 70base: 5'-GGT GAA AAC ACC GCA GCA TGT CAA GAT CAC AGA TTT TGG GC***G*** GGC CAA ACT GCT GGG TGC GGA AGA GAA A-3' (SEQ ID NO: 12) Mismatch 70base: 5'-GGT GAA AAC ACC GCA GCA TGT CAA GAT CAC AGA TTT TGG GC***T*** GGC CAA ACT GCT GGG TGC GGA AGA GAA A-3' (SEQ ID NO: 13)

**Standard method**

AuNPs@WT and AuNPs@MUT were mixed in a UV micro cuvette with PBS and NaCl. Finally, 2.5 µL of [Analyte] = 1 µM solution was added to the mixture, to reach a concentration of 5 nM. The hybridization process was followed by UV-Vis spectroscopy.

The assay is unable to detect long sequences. We speculate that the hybridization process is inhibited due to the secondary structure of match and mismatch sequences.

**Pre-Incubation Method**

To check whether secondary structure of match and mismatch inhibits particles aggregation, we then redesigned the assay in a way to promote the hybridization of the long sequence with the DNA-AuNP probe before addition of a second batch of nanoparticles. In doing so, the 70base analyte was preincubated (5 nM) with one type of AuNP probes in eppendorf upon rolling. After one hour of incubation, the other type of AuNP was added and again left on the roller mixer. The hybridization process was studied by UV-Vis spectroscopy. To confirm particles stability at mixing conditions, a control experiment with no analyte was carried out. After 3 hours of incubation, the differences in the colour between the match, mismatch and control experiments are clearly visible by naked eye.

To further confirm our observation by UV-Vis, DLS measurements were performed to evaluate the size of the aggregates during the hybridization process.

The mean diameter of the aggregates at different experimental steps was determined. It was found that the size of aggregates increases with the time only in the presence of the match sequence. For the mismatch, however, a slight increase was observed but at 3h; and in the control experiment, it was observed that the particles remained stable over entire experimental time.

We further checked if it is possible to detect lower concentrations (0.1 nM) of 70base analyte using pre-incubation method. The detection, indeed, was possible and the selectivity between the match and the mismatch was clear. However, with decreasing the concentration of the analyte the detection time increases.

The next step was to detect even longer DNA sequences. Experiments with analyte sequences of 140 bases were carried out using the pre-incubation method. The concentration of analyte was 5 nM.

We observed that after 2 hours of incubation there was a clear difference between the match and the mismatch sequence. While in the presence of match, the NPs are completely aggregated in the presence of mismatch the NPs remain stable.

## dsDNA 23base Detection - Thermal Denaturalization

The thermal denaturalization of dsDNA and the detection of ssDNA was studied. A kinetic study was performed using dsDNA as analyte, before the thermal treatment. A slight increase in the aggregation degree was observed. Then, in the thermobath, the solution was heated for 5 min to 70°C. After that, the hybridization process was recorded by UV-Vis and the aggregation degree was calculated.

The aggregation degree increases after the thermal denaturalization, but it is not so high as in the case of ssDNA detection. The DNA rehybridization process was found to compete with the hybridization with the capture probes.

## dsDNA 23base Detection - Blocking Probe

Further work was carried out to detect a single-base mutation in dsDNA in which one of the ssDNA sequences carries mutation. Thus, by following the PCR concept, it is necessary to dehybridize dsDNA and then inhibit renaturalization by using a short ssDNA sequence (blocking probe) that is complementary to mutation-free ssDNA (antisense analyte). Note that in this scenario, the blocking probe sequences are also complementary to capture probe (WT and MUT) anchored to the surface of nanoparticles. The following blocking probe sequences were selected, assigned as BP1 and BP2:

BP1: 5'GCG GGC CAA AC-3' (SEQ ID NO: 14)
BP2: 5'CAC AGA TTT TGG-3' (SEQ ID NO: 15)

First we confirmed that AuNPs remain stable in the presence of dsDNA (Analyte == Anti-Analyte) and aggregates in the presence of ssDNA (Analyte - Match or Mismatch). Then, we tested the ability of the blocking probe sequences to inhibit the formation of dsDNA (Analyte == Anti-Analyte). The Anti-Analyte sequence was incubated with either BP1 or BP2, as well as with the mixture of both. Then, analyte sequence was added which is complementary to Anti-Analyte. After a period of 15 min of incubation, AuNPs@WT and AuNPs@MUT were added and the hybridization kinetics were followed by UV-Vis. The concentration of Blocking Probe, Anti-Analyte and Analyte was 5 nM.

We expected to observe the displacement of Blocking Probes by Analyte and formation of dsDNA that, in turn, would inhibit particle aggregation. The results, however, show that this is not the case. The mixture of BP1 and BP2 can efficiently block Anti-Analyte, letting the Analyte aggregate AuNPs@WT and AuNPs@MUT. Interestingly, BP2 blocks Anti-Analyte more efficiently than BP1 for both Match and Mismatch.

The results showed that blocking Probes bind strongly to Anti-Analyte and subsequent addition of Analyte does not displace the blocking probes. In order to investigate whether anti-analyte added to the mixture containing Blocking Probes and Analyte (non complemetary) would bind faster to short Blocking Probes or long Analyte, experiments were performed for the match and the mismatch sequence, in a concentration of Blocking Probe, Anti-Analyte and Analyte of 5 nM.

Carrying out the experiment in this competitive way, a slight difference in the aggregation degree was observed between the match and the mismatch. The best blocking strategy was found to be the use of short oligonucleotides. The blocking capability of BP2 was found to be better than BP1.

Next, addition of blocking probes to dsDNA accompanied by thermal treatment was investigated to see if Blocking Probes can inhibit dsDNA formation once the temperature decreases. First, we formed dsDNA by mixing Analyte (match and mismatch) with the Anti-Analyte. Next, we added Blocking Probe in PBS/NaCl. The solution was heated to 65°C in the thermobath. After 10 min of heating, this solution was mixed with the AuNPs, followed UV-Vis recording. We observed that the aggregation of the particles in the presence and absence of BP was very similar for match and mismatch sequence.

**Blocking Probe Tritration**

As observed above, the Blocking Probes can block formation of dsDNA, facilitating detection of the mutation. Following this observation one can reason that Blocking Probes may perform even better at higher concentrations. However, we observed that Blocking Probe induce aggregation of the gold nanoparticles *in the absence of the analyte sequence.* Without wishing to be bound by any particular theory, the present inventors believe that the blocking probe, when bound to the capture probe on the nanoparticle's surface may decrease the electrostatic/steric repulsion between the nanoparticles, thereby facilitating non-specific aggregation. It is estimated that the concentration of oligonucleotides on the surface of the nanoparticles (WR or MUT) was 20 nM, which corresponds to 1500 ssDNA per nanoparticle. A concentration range of Blocking Probe was therefore selected, which spans 1 to 100 nM, so as to investigate the BP concentration below, through and above the concentration of the capture probes.

We observed that with the increase the concentration of Blocking Probes the colloidal stability of the nanoparticles decreases. Interestingly, at 20 nM of Blocking Probes, we observed an inflection point that corresponds to the total concentration of the capture probe on the particles surface. At even higher concentrations of Blocking Probe, the non-specific aggregation dominates. Therefore, a concentration of Blocking Probe up to 5 nM was selected as optimal under these conditions.

**Selectivity Induced by Blocking Probe**

Finally, we studied the effect of the concentration of Blocking Probe on the selectivity of the assay toward single-base mutation. The AuNPs coated with Capture Probes were hybridized with the Blocking Probes sequences in a concentration range between 0.5 and 5 nM.

The presence of blocking probes improves the selectivity of the assay toward single base mutation detection. We observed that with the increase BP concentration up to 3 nM the selectivity improves but further increases of the BP leads poorer specificity. The optimal concentration of 3 nM corresponds to ~6 Blocking Probes per capture probe. In accordance with present invention, where blocking probe is employed, the ratio of blocking probe to capture probe may in some cases be in the range 2-10, for example around 5-7, in particular around 6.

***Example 10 - Further targets and probe sequences***

Exemplary probes for detection of mutant BRAF are described in Example 1. Further examples of gene sequences that may serve as target analytes in accordance with the present invention include the cancer-related genes EGFR (NCBI Gene ID: 1956) and BRCA1 (NCBI Gene ID: 672). In particular, probes may be for detection of EGFR mutants L858R or T790M. Examples of EGFR L858R detection probes are shown below (SEQ ID NOs: 16-22):

*EGFR L858R 140mer WT (MM)*

5'-GGT-GAA-AAC-ACC-GCA-GCA-TGT-CAA-GAT-CAC-AGA-TTT-TGG-GC*T*-GGC-CAA-ACT-GCT-GGG-
TGC-GGA-AGA-GAA-AGA-ATA-CCA-TGC-AGA-AGG-AGG-CAA-AGT-GCC-TAT-CAA-GTG-GAT-GGC-
ATT-GGA-ATC-AAT-TTT-ACA-CAG-AAT-CT-3'

*EGFR L858R 140mer MUT (M)*

5'-GGT-GAA-AAC-ACC-GCA-GCA-TGT-CAA-GAT-CAC-AGA-TTT-TGG-GC*G*-GGC-CAA-ACT-GCT-GGG-
TGC-GGA-AGA-GAA-AGA-ATA-CCA-TGC-AGA-AGG-AGG-CAA-AGT-GCC-TAT-CAA-GTG-GAT-GGC-
ATT-GGA-ATC-AAT-TTT-ACA-CAG-AAT-CT-3'

*EGFR L858R 70mer WT (MM)*

5'-GGT-GAA-AAC-ACC-GCA-GCA-TGT-CAA-GAT-CAC-AGA-TTT-TGG-GC**T**-GGC-CAA-ACT-GCT-GGG-TGC-GGA-AGA-GAA-A-3'

*EGFR L858R 70mer MUT (M)*

5'-GGT-GAA-AAC-ACC-GCA-GCA-TGT-CAA-GAT-CAC-AGA-TTT-TGG-GC**G**-GGC-CAA-ACT-GCT-GGG-TGC-GGA-AGA-GAA-A-3'

*EGFR L858R 23mer WT (MM)*
5'-CAC-AGA-TTT-TGG-GC**T**-GGC-CAA-AC-3'

*EGFR L858R 23mer MUT (M)*      *EGFR L858R 23mer anti-MUT (antiM)*
5'-CAC-AGA-TTT-TGG-GC**G**-GGC-CAA-AC-3'     5'-GTT-TGG-CCC-GCC-CAA-AAT-CTG-TG-3'

As shown in Figure 14, both the 70-base and 140-base EGFR probes were able to effect mutant-analyte-specific nanoparticle agglomeration (as assessed by $Abs_{620}/Abs_{538}$ ratio) within 1 hour.
This provides a further demonstration of practical advantage because the length DNA sequences reflect the 150-165 nucleotide average length of plasma DNA (e.g. circulating tumour DNA) - see Underhill et al., PLoS Genet., 2016, 12(7): e1006162. Moreover, the analyte-specific agglomeration observed demonstrates the feasibility of detecting double stranded DNA sample by heat-induced denaturation (see Figure 15).

In certain cases the detection probes may be as disclosed in Sanromán-Iglesias et al., ACS Sens., 2016, Vol. 1, pp. 1110-1116 . These detection probes detect a SNP in the BRCA1 gene, an important indicator of increased risk to the development of breast and ovarian cancers (SEQ ID NOs: 23-26):

| Name | Oligonucleotide Sequences |
|---|---|
| Probe DNA conjugated on AuNP | |
| 1triplex (19 base) | HS- 3'-C$_6$-TTT-TTT-TTT-T*CT-TGT-TTT*-*C*-5' |
| 2Triplex) (20 base) | HS-5'-C$_6$-TTT-TTT-TTT-T<u>GA-TTT-TCT-TC</u>-3' |
| Target DNA for hybridization | |
| Match 119 base) | 5'-*GAA-CAA-AAG*-GAA-GAA.AAT-C-3' |
| Mismatch (19 base) | 5'-*GAA-CAA-AAG*-GAA-<u>TAA</u>-AAT-C-3' |

### Example 11 - Sample Preparation Unit (SPU)

The SPU is where whole blood is loaded before being separated into plasma by a microtrench system. The plasma is mixed with nanoparticle-probes (NP-probes) before being thermally separated into single strand (ss)DNA for efficient binding to the probe sequences. The SPU comprises the following components: sample loading device, SIMBAS plasma purification system, heating element and nanoparticle-probe reservoir.

**Sample loading device**

In order to activate the device, the patient presses down on a raised blister bulb sample area with a thumb or finger. The sample area contains a hollow bore needle protruding 1.6 mm from the main device deck and pressing it pricks the finger in a similar manner to lancing devices used routinely by diabetics for glucose testing. By obscuring the sample needle within the device we can minimize the distress caused to the patient, reduce accidental needle stick injuries and reduce contamination concerns of healthcare workers.

The device is packed under negative pressure, the resulting partial vacuum helps to facilitate blood draw into the device leading to a sample volume of 200-300 $\mu$l without the patient experiencing significant discomfort or seeing the blood sample itself.

**SIMBAS plasma purification system**

The blood sample enters a microtrench system where the red blood cells are removed to form plasma using the self-powered integrated microfluidic blood analysis system (SIMBAS) system (see Example 3 above). This system is more efficient and cost-effective than current membrane based blood separation technologies (see Figure 3).

**Heating element**

The plasma is heated because the DNA is double stranded (dsDNA) and therefore not very efficient at binding probes. The heat denatures the dsDNA to form single-stranded (ss)DNA, the same process as occurs in PCR. Also analogous to PCR, the ssDNA that contains the mutated sequence of interest binds preferentially to an excess of short complementary sequence-specific probes attached to the NPs.

Heating is carried out using a simple thin-film heating element embedded within the microchannel. Thin-film heating elements are much more rapid and efficient than bulk heating ($8°C\ s^{-1}$ cf. $1\text{-}3°C\ s^{-1}$) and can easily be powered on the device by button batteries.

Although flash heating is used to minimize the probability of protein denaturation, that has slower kinetics than DNA denaturation, some protein denaturation is likely to occur resulting in increased viscosity of the plasma. We have shown that this phenomenon can be largely overcome by diluting the plasma by 50% in the presence of Cibacron Blue, a protein absorbing dye which may be absorbed onto the microfludic channel after the SIMBAS module. We have shown that the Cibacron Blue treatment does not inhibit or remove the DNA from the plasma.

**NP-probe reservoir**

The resulting plasma (100-150 $\mu$l) enters a microfluidic channel where it is mixed with pre-loaded gold probe nanoparticles. We have optimized the sequence design of the nucleic acid probes for their ability to distinguish between single nucleotide mutated and wild type sequences as demonstrated by surface plasmon resonance studies (SPR) using a Biacore 3000 machine (see Figure 1).

The DNA probes were attached to the gold NPs via a thiol linkage and include a C18 spacer molecule to avoid non-specific binding of the probe to the gold surface. Each NP-probe contains 200-300 probes.

There are two distinct types of probe sequence (and NP-probes), one complementary to the normal (or wild type) sequence, and the other to a downstream (or upstream) sequence containing the mutation to be detected (Figure 6C). The patient ssDNA (the analyte) forms a bridge between the two probes bringing the NPs together to form an agglomerate. The distance between NPs can be controlled by modifying the design of the probes.

Although the WT sequence DNA can also bind to the upstream probe it cannot bind the mutated probe and therefore does not form an agglomerate (compare Figures 6A and 6B). We have demonstrated the ability of this system to distinguish between WT and single-nucleotide mutations down to a concentration of 10.8 fmol (see Sanromán-Iglesias et al., ACS Sens., 2016, Vol. 1, pp. 1110-1116).

In order further to optimise the analyte-induced nanoparticle-probe agglomeration, an anti-fouling solution may be employed to mitigate against plasma protein-induced blocking of agglomeration. Proteins from whole blood and/or plasma may coat the detection nanoparticles tending to inhibit the binding of analyte to nanoparticle-probe and subsequent agglomeration. One example of an anti-fouling solution is Optodex®. The OptoDex® platform is a surface engineering technology developed by the Centre Suisse d'Electronique et de Microtechnique (CSEM) that integrates technologies from material science, surface chemistry, and biochemistry, and includes novel materials, controllable and well characterized surface chemistries for biomolecule attachment and surface passivation.

***Example 12 - Signal Amplification Zone***

As an alternative to the signal amplification based on release of signal nanoparticles from micropores, which is described in Examples 7 and 8 above, the present inventors sought to achieve greater efficiency of signal release by employing a thermoresponsive microsphere-based signal reservoir. In particular, the use of microspheres addresses the problem of release of signal nanoparticles from micropores following breakdown of the thermoresponsive film that coats the micropore openings. The present inventors have found that, under certain conditions, release of signal nanoparticles from micropores was < 5% of the total that had been loaded into the micropores. By contrast, release of signal means from thermoresponsive microspheres following heat-induced disruption of the microsphere shell is estimated to be > 80% of the total that had been loaded into the microspheres:

| Release of signal means from signal reservoir | |
|---|---|
| Micropores | Microspheres |
| + | ++++ |

Alginate/agarose beads packed with signal nanoparticles were found to be significantly more leaky than microspheres based on polyelectrolytes, in particular, PHH/PSS bilayers. Therefore, the latter were selected as an optimal choice for signal reservoir for the signal means.

A schematic representation of the signal amplification zone is depicted in Figure 16. The detection nanoparticle agglomerates flow along the microchannels within the device until they encounter thermoresponsive microspheres (shown to the left in Figure 16) in the amplification zone.

The thermoresponsive microspheres (~10 $\mu$m diameter) are packed adjacent to much larger silica beads (~100 $\mu$m diameter) in a retaining chamber of the microfluidics channel (silica beads shown to the right in Figure 16). This arrangement similar to that found in chromatography ensures that the flow continues through the device without hindrance whilst bringing the detection nanoparticle agglomerates in close proximity to the thermoresponsive microspheres which are retained whilst intact.

The agglomerates are energized by an on-device red laser diode that delivers light at the resonant wavelength (i.e. 633 nm). The laser diode (1mW commercially available) is powered by the same button battery as the thin film heater.

The absorption of laser light at 633nm by agglomerated gold nanoparticles results in localised energy emission as plasmon resonance. These nanoheaters can raise the temperature well in excess of 40-50°C causing the disintegration of the thermoresponsive microspheres.

Although free-floating probe nanoparticles can also absorb light, this energy is less than 1% of that absorbed by agglomerated nanoparticles. There is little absorption by other components such as the plastics of the microfluidics device or the plasma itself or contaminating blood which has absorption maxima of at 434nm and 414nm for Hb and HbO2 respectively.

The thermoresponsive microspheres are composed of 5 x bilayer PSS/PAH using micelle co-precipitation $CaCO_3$ encapsulating ~$10^9$ molecules of signal molecules (e.g. strepavidin). This arrangement leads to massive amplification of the signal.

Signal molecules released by disrupted microspheres (and non-agglomerated probe-NPs) travel through the silica beads towards the test signal zone.

Examples of signal-loaded microspheres that have been tested include: PSS/PAH bilayer microcapsules loaded with the enzyme horse radish peroxidase (HRP) and PSS/PAH bilayer microcapsules loaded with dextran-FITC. Laser-induced release of the signal load has been demonstrated (results not shown). Furthermore, presence of agglomerated nanoparticles at the surface of microcapsules has been visualised by electron microscopy.

**Microcapsule production and packing in microchannels**

Thermoresponsive microspheres were produced essentially as described in Ambrosone et al., ACS Nano, 2016, Vol. 10(4), pp. 4828-4834 (see supplementary materials S2).

Poly(sodium 4-styrenesulfonate) (PSS, Mw ≈ 70 kDa, #243051), poly(allylamine hydrochloride) (PAH, Mw ≈ 56 kDa, #283223), calcium chloride dihydrate ($CaCl_2$, #223506), sodium carbonate ($Na_2CO_3$, #S7795), and poly(styrene)-block-poly(acrylic acid) (PSS-b-PAA, Mn ≈ 8700, #735892) were purchased from Sigma-Aldrich.

The PSS/PAH $CaCO_3$ microspheres (diameter 5-10 $\mu$m) were loaded with HRP, dextran-FITC or streptavidin as signal means. The multilayer polyelectolyte shell (e.g. 5-8 alternating layers of PSS and PAH) may be provide a positive or a negative charge on the outermost layer.

**Retaining chamber design**

The microfluidics retaining chamber addresses the problem of how to bring all components together (i.e. agglomerates and microspheres) whilst retaining flow through device. The retaining chamber comprises a microchannel packed with larger silica/glass beads (~50-100$\mu$m), these in turn retain the intact microspheres (~5-10$\mu$m) and the flow brings the agglomerates to the microspheres whilst allowing free-floating NPs to continue through device along with signal molecules released from disrupted microspheres.

Microsphere packing in cyclic olefin polymer (COP) channels was investigated. Glass beads of diameter 75$\mu$m were used to block the microchannel, thereby retaining the microspheres (5-10$\mu$m) in position (see Figure 18A). Using 8-layer microspheres terminating in a negative outermost layer, the microspheres were stable (not leaking dextran-FITC loaded signal) for at least 3 months and were retained in position. An alternative solution for clogging the COP channels was also tested: agarose beads of diameter 150$\mu$m.

Laser-induced release of dextran-FITC signal means from microspheres is depicted in Figure 18B.

### Example 13 - Test Signal Zone

Following release of the signal means from the signal reservoir (e.g. microspheres), the signal means flows to the test signal zone (e.g. a nitrocellulose strip) where the signal means is captured on a retention strip by means of a specific binding interaction, thereby resulting in a visible line. The test signal zone may employ standard components as used in commercially available lateral flow test kits (e.g. pregnancy test kits). In one example, the signal means may comprise streptavidin and the retention strip may comprise biotin.

### Example 14 - Integrated Device and Diagnostic Applications

In some embodiments, the present invention provides an integrated device comprising the separate, but functionally connected, modules:

1. Sample preparation unit (SPU). Whole blood is loaded onto the SPU where it is separated into plasma by a microtrench system. The plasma is mixed with nanoparticle-probes (NP-probes) before being thermally separated into single strand (ss)DNA for efficient binding to the probe sequences.

2. Amplification zone. Specific binding of the mutated ssDNA sequence to two complementary bridging DNA probes results in their specific agglomeration. Agglomeration brings the NPs in close proximity to each other, and when energized by a laser diode results in plasmon resonance converting the light energy into localized thermal energy. This thermal energy disrupts thermoresponsive microspheres that encapsulate tens of millions of signal molecules corresponding to a massive amplification of the signal.

3. Test signal. The released signal molecules travel along a nitrocellulose strip until captured by a test strip resulting in a visible line.

A schematic illustration of the integrated device is depicted in Figure 19. The device is a hybrid microfluidics-lateral flow device, the microfluidics section consisting of the SPU and the amplification zone is powered by Degas flow via specially designed micropumps as a result of the device being packaged under negative pressure. The lateral flow test signal region is powered by capillary force of the liquid leaving the microfluidics section via a reservoir arrangement. Button-batteries power the on-device laser diode and heating elements. The SPU contains an interchangeable NP-probe module that allows for testing different biomarker sequences within a standardized test housing to minimize manufacturing costs.

The detection of EGFR mutations in NSCLC patients for example is indicative of their response to tyrosine kinase inhibitors (TKIs) such as Gefitinib, erlotinib, brigatinib and lapatinib. The major mutations are the L858R mutation present in up 90% of EGFR-mutated NSCLC cases, and exon 19 deletions present in ~40% of cases. Both mutations may be detected by the device of the present invention. Current licensed companion diagnostic technologies for EGFR mutation testing are PCR-based and rely upon invasively-obtained biopsies which is sub-optimal for patient and clinician convenience.

In the case of NSCLC, the latest ESMO guidelines recommend the monitoring of the appearance of the T790M mutation as an indicator of emerging resistance to TKI-based therapies and consequent switching to a second line therapy (Osimertinib). Current follow-up procedures for NSCLC patients requires hospital visits and the use of imaging techniques such as CT scans. The device of the present invention offers a simpler alternative that may be realised in a local healthcare centre setting.

The specific embodiments described herein are offered by way of example, not by way of limitation. Any subtitles herein are included for convenience only, and are not to be construed as limiting the disclosure in any way.

### Claims

**1.** A method for detecting the presence or absence of a target analyte in a test sample, comprising the steps of:

i) providing a plurality of detector nanoparticles comprising a first detector nanoparticle functionalised with a first probe specific for a first region of a target analyte and a second detector nanoparticle functionalised with a second probe specific for a second region of said target analyte; and

ii) contacting said detector nanoparticles with a test sample under conditions suitable for the binding of the specific probes to the target analyte, wherein target analyte-induced agglomeration of the detector nanoparticles permits the release of a detectable signal means from a signal reservoir by breakdown of a partition closing the signal reservoir, and wherein the partition is a thermoresponsive polymer layer and the agglomerated detector nanoparticles direct a heat transfer sufficient to cause at least partial thermal breakdown of said thermoresponsive polymer layer, thereby releasing the detectable signal means, and wherein the heat transfer is triggered by irradiation of the agglomerated detector nanoparticles with a source of electromagnetic radiation.

2. The method according to claim 1, wherein the signal reservoir comprises one or more microspheres having a core comprising said signal means and a shell enclosing said core, and wherein the shell is said partition.

3. The method according to any one of claims 1 and 2, wherein the target analyte is a nucleic acid.

4. The method according to any one of claims 1 to 3, wherein:

(i) the thermoresponsive polymer comprises at least one polymer selected from the group consisting of: poly(styrene sulfonate) (PSS), poly(diallyldimethylammonium chloride) (PDADMAC), poly(allylamine) (PAH), poly(N-isopropylacrylamide), poly[2-(dimethylamino)ethyl methacrylate] (pDMAEMA), hydroxypropylcellulose, poly(vinylcaprolactame) and polyvinyl methyl ether; and/or
(ii) irradiation of the agglomerated detector nanoparticles produces a local temperature increase of at least 20°C, 40°C, or 60°C; and/or
(iii) the source of electromagnetic radiation is a laser diode or a light emitting diode (LED); and/or
(iv) detector nanoparticle agglomerates localise to the partition following agglomeration.
(v) the detector nanoparticles comprise a core of metal atoms.
(vi) wherein the signal means comprises streptavidin, an antibody, an enzyme, a fluorophore, a dye, one or more quantum dots, one or more latex beads, or is a plurality of signal nanoparticles.

5. The method according to any one of the preceding claims, wherein the signal reservoir comprises a plurality of said microspheres, and wherein:

(i) the average diameter of the microspheres is in the range 1 to 20 $\mu$m, optionally 5 to 10 $\mu$m; and/or
(ii) the surface layer of the shell is negatively charged or is positively charged.

6. The method according to claim 3, wherein the detector nanoparticle probes are oligonucleotides complementary to first and second regions of the target nucleic acid.

7. The method according to claim 3, wherein the target nucleic acid has a sequence which is a variant of a wild-type sequence, and said first or second probes bind to the variant sequence in preference to the wild-type sequence.

8. The method according to claim 7, wherein the variant sequence comprises a single nucleotide polymorphism (SNP) and the first or second detector nanoparticle probe hybridises to a portion of the target nucleic acid comprising the SNP position.

9. The method according to claim 7 or claim 8, wherein the variant sequence comprises a mutation associated with cancer, optionally wherein said mutation is selected from the group consisting of: a single nucleotide change, a deletion, an insertion or a sequence translocation.

10. The method according to claim 9, wherein the mutation is in a gene selected from the group consisting of: human epidermal growth factor receptor (EGFR) of NCBI Gene ID: 1956; human Breast cancer 1 early onset (BRCA1) of NCBI Gene ID: 672; the human BRAF gene of NCBI Gene ID: 673; and the human KRAS proto-oncogene of NCBI Gene ID: 3845.

11. A device for detecting the presence or absence of a target analyte in a test sample, comprising:

i) a detection compartment, containing a plurality of detector nanoparticles comprising a first detector nanoparticle functionalised with a first probe specific for a first region of a target analyte and a second detector nanoparticle functionalised with a second probe specific for a second region of said target analyte; and
ii) a signal amplification zone containing a detectable signal means contained in a signal reservoir by a partition that is selectively disruptable following target analyte-induced agglomeration of the detector nanoparticles, wherein the signal amplification zone further comprises an integrated source of electromagnetic radiation configured to provide energy at the resonant wavelength of the detector nanoparticles, and wherein the partition comprises a thermoresponsive polymer layer.

12. The device according to claim 11, wherein the signal reservoir comprises one or more microspheres having a core comprising said signal means and a shell enclosing said core, and wherein the shell is said partition.

13. The device according to any one of claims 11 and 12, wherein the detection compartment further contains a plurality of blocking probes specific for the antisense strand of a target nucleic acid.

14. Use of a device as defined in any one of claims 11 to 13 in a method of:

diagnosis or prognosis of a mammalian subject, wherein said test sample is a biological sample that has been obtained from said subject; or
detection of a bacterial, parasitic, or other biological contaminant, wherein said test sample is an environmental sample.

15. A kit comprising:

i) a device having:

a sample inlet;
a lysis compartment comprising a heating element;
an integrated source of electromagnetic radiation;
a plurality of thermoresponsive microspheres having a core comprising signal means and a shell enclosing the core, wherein the shell comprises a thermoresponsive polymer; and
a signal display region comprising a retention strip to capture the signal means following release from the microspheres;

optionally,
ii) one or more populations of a blocking probe capable of specific binding to the antisense strand of a target nucleic acid; and
iii) one or more populations of a plurality of detector nanoparticles functionalised with a first probe specific for a first region of the sense strand of said target nucleic acid and a second probe specific for a second region of said target nucleic acid.

**Patentansprüche**

1. Verfahren zum Detektieren der Gegenwart oder Abwesenheit eines Zielanalyten in einer Testprobe, umfassend die folgenden Schritte:

i) das Bereitstellen einer Vielzahl von Detektor-Nanopartikeln, die ein erstes Detektor-Nanopartikel, das mit einer ersten, für eine erste Region eines Zielanalyten spezifischen Sonde funktionalisiert ist, und ein zweites Detektor-Nanopartikel umfassen, das mit einer zweiten, für eine zweite Region des Zielanalyten spezifischen Sonde funktionalisiert ist; und
ii) das Kontaktieren der Detektor-Nanopartikel mit einer Testprobe unter Bedingungen, die für die Bindung der spezifischen Sonden an den Zielanalyten geeignet sind, wobei Zielanalyt-induzierte Agglomeration der Detektor-Nanopartikel die Freisetzung eines detektierbaren Signalmittels aus einem Signalreservoir mittels Zusammenbruchs einer Trennwand, die das Signalreservoir verschließt, erlaubt und wobei die Trennwand eine thermoreaktive Polymerschicht ist und die agglomerierten Detektor-Nanopartikel eine Wärmeübertragung steuern, die ausreicht, um einen zumindest teilweisen thermischen Zusammenbruch der thermoreaktiven Polymerschicht herbeizuführen, wodurch das detektierbare Signalmittel freigesetzt wird, und wobei die Wärmeübertragung durch Bestrahlung der agglomerierten Detektor-Nanopartikel mit einer Quelle elektromagnetischer Strahlung ausgelöst wird.

2. Verfahren nach Anspruch 1, wobei das Signalreservoir ein oder mehrere Mikrokügelchen umfasst, die einen Kern, der das Signalmittel umfasst, und eine Hülle, die den Kern umschließt, aufweisen, und wobei die Hülle die Trennwand ist.

3. Verfahren nach einem der Ansprüche 1 und 2, wobei der Zielanalyt eine Nucleinsäure ist.

4. Verfahren nach einem der Ansprüche 1 bis 3, wobei:

(i) das thermoreaktive Polymer zumindest ein Polymer umfasst, das aus der aus: Poly(styrolsulfonat) (PSS), Poly(diallyldimethylammoniumchlorid) (PDADMAC), Poly(allylamin) (PAH), Poly(N-isopropylacrylamid), Poly[2-(dimethylamino)ethylmethacrylat] (pDMAEMA), Hydroxypropylcellulose, Poly(vinylcaprolactam) und Polyvinylmethylester bestehenden Gruppe ausgewählt ist; und/oder
(ii) die Bestrahlung der agglomerierten Detektor-Nanopartikel einen lokalen Temperaturanstieg von zumindest 20 °C, 40 °C oder 60 °C erzeugt; und/oder
(iii) die Quelle elektromagnetischer Strahlung eine Laserdiode oder eine lichtemittierende Diode (LED) ist; und/oder
(iv) Detektor-Nanopartikelagglomerate sich nach der Agglomeration an der Trennwand ansammeln;
(v) die Detektor-Nanopartikel einen Kern aus Metallatomen umfassen.
(vi) das Signalmittel Streptavidin, einen Antikörper, ein Enzym, einen Fluorophor, einen Farbstoff, einen oder mehrere Quantenpunkte oder ein oder mehrere Latexkügelchen umfasst oder eine Vielzahl von Signal-Nanopartikeln ist.

5. Verfahren nach einem der vorangegangenen Ansprüche, wobei das Signalreservoir eine Vielzahl der Mikrokügelchen umfasst und wobei:

(i) der mittlere Durchmesser der Mikrokügelchen im Bereich von 1 bis 20 $\mu$m gegebenenfalls 5 bis 10 $\mu$m, liegt; und/oder
(ii) die Oberflächenschicht der Hülle negativ geladen oder positiv geladen ist.

6. Verfahren nach Anspruch 3, wobei die Detektor-Nanopartikel-Sonden Oligonucleotide sind, die zu einer ersten und einer zweiten Region der Zielnucleinsäure komplementär sind.

7. Verfahren nach Anspruch 3, wobei die Zielnucleinsäure eine Sequenz aufweist, die eine Variante einer Wildtypsequenz ist, und die erste oder die zweite Sonde gegenüber der Wildtypsequenz bevorzugt an die Sequenzvariante bindet.

8. Verfahren nach Anspruch 7, wobei die Sequenzvariante einen Einzelnucleotid-Polymorphismus (SNP) umfasst und die erste oder die zweite Detektor-Nanopartikel-Sonde an einen Abschnitt der Zielnucleinsäure hybridisiert, die die SNP-Position umfasst.

9. Verfahren nach Anspruch 7 oder 8, wobei die Sequenzvariante eine mit Krebs assoziierte Mutation umfasst, wobei die Mutation gegebenenfalls aus der aus einer Einzelnucleotidänderung, einer Deletion, einer Insertion und einer Sequenztranslokation bestehenden Gruppe ausgewählt ist.

10. Verfahren nach Anspruch 9, wobei die Mutation in einem Gen liegt, das aus der aus menschlichem Epidermis-Wachstumsfaktorrezeptor (EGFR) des NCBI-Gens ID: 1956; menschlichem Early-Onset-Brustkrebs 1 (BRCA1) des NCBI-Gens ID: 672; dem menschlichen BRAF-Gen des NCBI-Gens ID: 673; und dem menschlichen KRAS-Protoonkogen des NCBI-Gens ID: 3845; bestehenden Gruppe ausgewählt ist.

11. Vorrichtung zum Detektieren der Gegenwart oder Abwesenheit eines Zielanalyten in einer Testprobe, umfassend:

i) ein Detektionskompartiment, das eine Vielzahl von Detektor-Nanopartikeln enthält, die ein erstes Detektor-Nanopartikel, das mit einer ersten, für eine erste Region eines Zielanalyten spezifischen Sonde funktionalisiert ist, und ein zweites Detektor-Nanopartikel umfassen, das mit einer zweiten, für eine zweite Region des Zielanalyten spezifischen Sonde funktionalisiert ist; und
ii) eine Signalamplifizierungszone, die ein detektierbares Signalmittel enthält, das in einem Signalreservoir mit einer Trennwand enthalten ist, die nach Zielanalytinduzierter Agglomeration der Detektor-Nanopartikel selektiv zerstörbar ist, wobei die Signalamplifizierungszone weiters eine integrierte Quelle elektromagnetischer Strahlung umfasst, die so konfiguriert ist, dass sie Energie mit der Resonanzwellenlänge der Detektor-Nanopartikel bereitstellt, und wobei die Trennwand eine thermoreaktive Polymerschicht umfasst.

**12.** Verfahren nach Anspruch 11, wobei das Signalreservoir ein oder mehrere Mikrokügelchen umfasst, die einen Kern, der das Signalmittel umfasst, und eine Hülle, die den Kern umschließt, aufweisen, und wobei die Hülle die Trennwand ist.

**13.** Vorrichtung nach einem der Ansprüche 11 und 12, wobei das Detektionskompartiment weiters eine Vielzahl von Blockiersonden enthält, die für den Antisense-Strang einer Zielnucleinsäure spezifisch sind.

**14.** Verwendung einer Vorrichtung nach einem der Ansprüche 11 bis 13 in einem Verfahren zur:

Diagnose oder Prognose eines Säugetierindividuums, wobei die Testprobe eine biologische Probe ist, die aus dem Individuum erhalten wurde; oder
Detektion einer bakteriellen, parasitischen oder anderen biologischen Verunreinigung, wobei die Testprobe eine Umweltprobe ist.

**15.** Set, das Folgendes umfasst:

i) eine Vorrichtung, die Folgendes aufweist:

einen Probeneinlass;
ein Lysekompartiment, das ein Heizelement umfasst;
eine integrierte Quelle elektromagnetischer Strahlung;
eine Vielzahl thermoreaktiver Mikrokügelchen, die einen Kern, der ein Signalmittel umfasst, und eine Hülle, die den Kern umschließt, aufweisen, wobei die Hülle ein thermoreaktives Polymer umfasst; und
eine Signalanzeigeregion, die einen Retentionsstreifen umfasst, um das Signalmittel nach der Freisetzung aus den Mikrokügelchen einzufangen;

ii) gegebenenfalls eine oder mehrere Populationen einer Blockiersonde, die in der Lage zu spezifischer Bindung an den Antisense-Strang einer Zielnucleinsäure sind; und
iii) eine oder mehrere Populationen einer Vielzahl von Detektor-Nanopartikeln, die mit einer ersten, für eine erste Region des Sense-Strangs der Zielnucleinsäure spezifischen Sonde und einer zweiten, für eine zweite Region der Zielnucleinsäure spezifischen Sonde funktionalisiert sind.

## Revendications

**1.** Procédé de détection de la présence ou de l'absence d'un analyte cible dans un échantillon d'essai, comprenant les étapes de :

i) fourniture d'une pluralité de nanoparticules de détection comprenant une première nanoparticule de détection fonctionnalisée avec une première sonde spécifique à une première région d'un analyte cible et une seconde nanoparticule de détection fonctionnalisée avec une seconde sonde spécifique à une seconde région dudit analyte cible ; et
ii) mise en contact desdites nanoparticules de détection avec un échantillon d'essai dans des conditions appropriées pour la liaison des sondes spécifiques à l'analyte cible, dans lequel l'agglomération induite par l'analyte cible des nanoparticules de détection permet la libération d'un moyen de signal détectable à partir d'un réservoir de signaux par décomposition d'une cloison fermant le réservoir de signaux, et dans lequel la cloison est une couche de polymère thermosensible et les nanoparticules de détection agglomérées conduisent à un transfert de chaleur suffisant pour provoquer une décomposition thermique au moins partielle de ladite couche de polymère thermosensible, en libérant ainsi le moyen de signal détectable, et dans lequel le transfert de chaleur est déclenché par irradiation des nanoparticules de détection agglomérées avec une source de rayonnement électromagnétique.

**2.** Procédé selon la revendication 1, dans lequel le réservoir de signaux comprend une ou plusieurs microsphères comportant un noyau comprenant ledit moyen de signal et une enveloppe renfermant ledit noyau, et dans lequel l'enveloppe est ladite cloison.

**3.** Procédé selon l'une quelconque des revendications 1 et 2, dans lequel l'analyte cible est un acide nucléique.

**4.** Procédé selon l'une quelconque des revendications 1 à 3, dans lequel :

(i) le polymère thermosensible comprend au moins un polymère choisi dans le groupe constitué de : poly(sulfonate de styrène) (PSS), poly(chlorure de diallyldiméthylammonium) (PDADMAC), poly(allylamine) (PAH), poly(N-isopropylacrylamide), poly[méthacrylate de 2-(diméthylamino)éthyle] (pDMAEMA), hydroxypropylcellulose, poly(vinylcaprolactame) et éther polyvinylméthylique ; et/ou

(ii) l'irradiation des nanoparticules de détection agglomérées produit une augmentation de température locale d'au moins 20 °C, 40 °C ou 60 °C ; et/ou

(iii) la source de rayonnement électromagnétique est une diode laser ou une diode électroluminescente (DEL) ; et/ou

(iv) les agglomérats de nanoparticules de détection sont localisés sur la cloison suite à l'agglomération.

(v) les nanoparticules de détection comprennent un noyau d'atomes de métal.

(vi) dans lequel le moyen de signal comprend de la streptavidine, un anticorps, une enzyme, un fluorophore, un colorant, un ou plusieurs points quantiques, une ou plusieurs billes de latex, ou est une pluralité de nanoparticules de signal.

**5.** Procédé selon l'une quelconque des revendications précédentes, dans lequel le réservoir de signaux comprend une pluralité desdites microsphères, et dans lequel :

(i) le diamètre moyen des microsphères est dans la plage de 1 à 20 $\mu$m, facultativement de 5 à 10 $\mu$m ; et/ou

(ii) la couche de surface de l'enveloppe est chargée négativement ou est chargée positivement.

**6.** Procédé selon la revendication 3, dans lequel les sondes de nanoparticule de détection sont des oligonucléotides complémentaires des première et seconde régions de l'acide nucléique cible.

**7.** Procédé selon la revendication 3, dans lequel l'acide nucléique cible comporte une séquence qui est un variant d'une séquence de type sauvage, et lesdites première ou seconde sondes se lient à la séquence de variant préférentiellement à la séquence de type sauvage.

**8.** Procédé selon la revendication 7, dans lequel la séquence de variant comprend un polymorphisme mononucléotidique (PMN) et la première ou la seconde sonde de nanoparticule de détection s'hybride à une partie de l'acide nucléique cible comprenant la position du PMN.

**9.** Procédé selon la revendication 7 ou la revendication 8, dans lequel la séquence de variant comprend une mutation associée à un cancer, facultativement dans lequel ladite mutation est choisie dans le groupe constitué de : une modification d'un seul nucléotide, une délétion, une insertion ou une translocation de séquences.

**10.** Procédé selon la revendication 9, dans lequel la mutation se situe dans un gène choisi dans le groupe constitué de : récepteur du facteur de croissance de l'épiderme (EGFR) humain d'ID de gène NCBI : 1956 ; cancer du sein humain 1 à début précoce (BRCA1) d'ID de gène NCBI : 672 ; le gène BRAF humain d'ID de gène NCBI : 673 ; et le proto-oncogène KRAS humain d'ID de gène NCBI : 3845.

**11.** Dispositif de détection de la présence ou de l'absence d'un analyte cible dans un échantillon d'essai, comprenant :

i) un compartiment de détection, contenant une pluralité de nanoparticules de détection comprenant une première nanoparticule de détection fonctionnalisée avec une première sonde spécifique à une première région d'un analyte cible et une seconde nanoparticule de détection fonctionnalisée avec une seconde sonde spécifique à une seconde région dudit analyte cible ; et

ii) une zone d'amplification de signal contenant un moyen de signal détectable contenue dans un réservoir de signaux par une cloison qui est sélectivement dissociable suite à l'agglomération induite par l'analyte cible des nanoparticules de détection, dans lequel la zone d'amplification de signal comprend en outre une source intégrée de rayonnement électromagnétique configurée pour fournir de l'énergie à la longueur d'onde de résonance des nanoparticules de détection, et dans lequel la cloison comprend une couche de polymère thermosensible.

**12.** Dispositif selon la revendication 11, dans lequel le réservoir de signaux comprend une ou plusieurs microsphères comportant un noyau comprenant ledit moyen de signal et une enveloppe renfermant ledit noyau, et dans lequel l'enveloppe est ladite cloison.

13. Dispositif selon l'une quelconque des revendications 11 et 12, dans lequel le compartiment de détection contient en outre une pluralité de sondes de blocage spécifiques au brin antisens d'un acide nucléique cible.

14. Utilisation d'un dispositif selon l'une quelconque des revendications 11 à 13 dans un procédé de :

diagnostic ou pronostic d'un sujet mammifère, dans lequel ledit échantillon d'essai est un échantillon biologique qui a été obtenu à partir dudit sujet ; ou
détection d'un contaminant bactérien, parasitaire ou autre contaminant biologique, dans lequel ledit échantillon d'essai est un échantillon environnemental.

15. Kit comprenant :

i) un dispositif comportant :

une entrée d'échantillon ;
un compartiment de lyse comprenant un élément chauffant ;
une source intégrée de rayonnement électromagnétique ;
une pluralité de microsphères thermosensibles comportant un noyau comprenant un moyen de signal et une enveloppe renfermant le noyau, dans lequel l'enveloppe comprend un polymère thermosensible ; et
une région d'affichage de signal comprenant une bande de rétention pour capturer le moyen de signal suite à la libération à partir des microsphères ;

facultativement,
ii) une ou plusieurs populations d'une sonde de blocage capable de se lier spécifiquement au brin antisens d'un acide nucléique cible ; et
iii) une ou plusieurs populations d'une pluralité de nanoparticules de détection fonctionnalisées avec une première sonde spécifique à une première région du brin sens dudit acide nucléique cible et une seconde sonde spécifique à une seconde région dudit acide nucléique cible.

Figure 1

A

B

Figure 2

A

B

C

D

Figure 3

A

B

C

EP 3 310 930 B1

Figure 4

Figure 5

Figure 6

Figure 7A

Figure 7B

Figure 7C

Figure 7D

Figure 7E

Figure 7F

Figure 7G

Figure 7H

Figure 7I

Figure 8

A B

C D

Figure 9

Figure 10

Figure 11

Figure 12

Figure 13

Figure 14

Figure 15

Figure 16

Figure 17

Figure 18A

Before Laser    After Laser

Figure 18B

Figure 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005118870 A
- WO 2007044025 A
- EP 1867990 A
- WO 8202772 A
- US 20040180369 A
- WO 9002205 A
- WO 9923258 A

### Non-patent literature cited in the description

- **DORIA et al.** *Sensors,* 2012, vol. 12, 1657-1687
- **SANROMÁN-IGLESIAS et al.** *ACS Appl. Mater. Interfaces,* 2015, vol. 7, 15692-15695
- **SANROMÁN-IGLESIAS et al.** *ACS Sens.,* 2016, vol. 1, 1110-1116
- **ZHOU et al.** *J Am Chem Soc,* 2010, vol. 26, 6932-6934
- **BUI et al.** *Anal Bioanal Chem,* 2007, vol. 388, 1185-1190
- **GOVOROV et al.** *Nanotoday,* 2007, vol. 2 (1), 30-38
- **Y.C. CAO ; R. JIN ; C. A. MIRKIN.** *Science,* 2002, vol. 297, 1536-1539
- Foundations of Genetic Algorithms. **GOLDBERG ; DEB.** Foundations of Genetic Algorithms. Morgan Kaufmann, 1991
- **SUKHAREV ; SEIDEMAN.** *Nano Lett,* 2006, vol. 6 (4), 715-719
- **ROUSSEL et al.** *Phys Rev Lett,* 2007, vol. 98, 114502
- **OWCZARZY et al.** *Biopolymers,* 1997, vol. 44 (3), 217-239
- **BERG ; VON HIPPEL.** *J Mol Biol,* 1987, vol. 193, 723-750
- **BAFFOU ; QUIDANT.** *Laser Photonics Rev,* 2013, vol. 7, 171-187
- **HOHENESTER ; TRÜGLER.** *Comput Phys Commun,* 2012, vol. 183, 370-381
- **BAFFOU et al.** *Appl Phys Lett,* 2009, vol. 94, 153109-153103
- **SPINDLER et al.** *Clin Cancer Res,* 2012, vol. 18, 1177-1185
- **DIMOV et al.** *Lab Chip,* 2011, vol. 11, 845-850
- **TURKEVICH et al.** *Discuss Faraday Soc,* 1951, vol. 11, 55-75
- **MIRKIN et al.** *Anal Chem,* 2006, vol. 7, 8313-8318
- **MAO et al.** *Anal. Chem,* 2009, vol. 81, 1660-1668
- **LI et al.** *J Phys Chem B.,* 2006, vol. 110 (32), 15755-15762
- **UNDERHILL et al.** *PLoS Genet.,* 2016, vol. 12 (7), e1006162
- **AMBROSONE et al.** *ACS Nano,* 2016, vol. 10 (4), 4828-4834